# EUROPÄISCHE PATENTSCHRIFT

(11) **EP 3 505 935 B1**
(45) Veröffentlichungstag und Bekanntmachung des Hinweises auf die Patenterteilung: **01.03.2023**
(21) Anmeldenummer: 18214997.1
(22) Anmeldetag: 21.12.2018
(51) Int. Cl.: G01N 33/569, G01N 33/543

(54) **VERFAHREN ZUR DIAGNOSE EINER BORNAVIRUS-INFEKTION**
METHOD FOR THE DIAGNOSIS OF A BORNA VIRUS INFECTION
PROCÉDÉ DE DIAGNOSTIC D'UNE INFECTION AUX VIRUS DE LA MALADIE DE BORNA

(30) Priorität: 29.12.2017 EP 17211138
(43) Veröffentlichungstag der Anmeldung: 03.07.2019
(73) Patentinhaber: Euroimmun Medizinische Labordiagnostika AG, 23560 Lübeck (DE)
(72) Erfinder: Lattwein, Erik, 23564 Lübeck (DE); Meyer, Wolfgang, 23689 Pansdorf (DE); Schlumberger, Wolfgang, 23627 Groß Grönau (DE); Janz, Julia, 22929 Schönberg (DE); Ott, Anthonina, 23847 Schiphorst (DE); Scheper, Thomas, 23919 Berkenthin (DE); Schmidt-Chanasit, Jonas, 10115 Berlin (DE); Tappe, Dennis, 20257 Hamburg (DE)

(56) Entgegenhaltungen:
- WO-A1-98/18457
- WO-A1-98/30238
- WO-A1-99/34216
- JP-A- H11 180 998
- US-A1- 2009 155 261
- US-A1- 2013 072 582
- Q. LI ET AL: "Detection and analysis of Borna disease virus in Chinese patients with neurological disorders", EUROPEAN JOURNAL OF NEUROLOGY, Bd. 16, Nr. 3, 1. März 2009 (2009-03-01), Seiten 399-403, XP055466461, GB ISSN: 1351-5101, DOI: 10.1111/j.1468-1331.2008.02516.x
- MATSUNAGA H ET AL: "Isotype analysis of human anti-Borna disease virus antibodies in Japanese psychiatric and general population", JOURNAL OF CLINICAL VIROLOGY, ELSEVIER, AMSTERDAM, NL, Bd. 43, Nr. 3, 1. November 2008 (2008-11-01), Seiten 317-322, XP025612281, ISSN: 1386-6532, DOI: 10.1016/J.JCV.2008.07.011 [gefunden am 2008-09-10]
- BERND HOFFMANN ET AL: "A Variegated Squirrel Bornavirus Associated with Fatal Human Encephalitis", NEW ENGLAND JOURNAL OF MEDICINE, THE - NEJM -, Bd. 373, Nr. 2, 9. Juli 2015 (2015-07-09), Seiten 154-162, XP055466467, US ISSN: 0028-4793, DOI: 10.1056/NEJMoa1415627
- Heng-Thay Chong ET AL: "Treatment of acute Nipah encephalitis with ribavirin", ANNALS OF NEUROLOGY., vol. 49, no. 6, 1 June 2001 (2001-06-01), pages 810-813, XP055705425, BOSTON, US ISSN: 0364-5134, DOI: 10.1002/ana.1062
- HORNIG M ET AL: "Absence of evidence for bornavirus infection in schizophrenia, bipolar disorder and major depressive disorder", MOLECULAR PSYCHIATRY, NATURE PUBLISHING GROUP UK, LONDON, vol. 17, no. 5, 31 January 2012 (2012-01-31), pages 486-493, XP037789777, ISSN: 1359-4184, DOI: 10.1038/MP.2011.179 [retrieved on 2012-01-31]
- IAN LIPKIN W ET AL: "Borna disease virus Fact and fantasy", VIRUS RESEARCH, vol. 162, no. 1, 31 December 2011 (2011-12-31), pages 162-172, XP028115835, ISSN: 0168-1702, DOI: 10.1016/J.VIRUSRES.2011.09.036 [retrieved on 2011-09-29]
- Dürrwald R. ET AL: "Meta-analysis of putative human Bornavirus...", , 31 December 2007 (2007-12-31), XP055948774, Retrieved from the Internet: URL:https://onlinelibrary.wiley.com/doi/10 .1002/rmv.530 [retrieved on 2022-08-03]
- MELZER NICO ET AL: "Limbic Encephalitis: Potential Impact of Adaptive Autoimmune Inflammation on Neuronal Circuits of the Amygdala", FRONTIERS IN NEUROLOGY, vol. 6, 3 August 2015 (2015-08-03), XP055948775, DOI: 10.3389/fneur.2015.00171
- DENNIS TAPPE ET AL: "Occupation-Associated Fatal Limbic Encephalitis Caused by Variegated Squirrel Bornavirus 1, Germany, 2013", EMERGING INFECTIOUS DISEASES, vol. 24, no. 6, 1 June 2018 (2018-06-01), pages 978-987, XP055705298, US ISSN: 1080-6040, DOI: 10.3201/eid2406.172027

## Beschreibung

Die vorliegende Erfindung betrifft insbesondere ein Verfahren zur Diagnose einer limbischen Encephalitis, Paraneoplastischen Syndromes (PNS), Encephaloymyelitis, Leukoencephalopathie, Retinitis oder Optikusatrophie umfassend den Schritt Nachweisen eines Mammalian 2 Bornavirus VSBV-1, umfassend den Schritt Nachweisen eines und Mammalian 1 Bornavirus BoDV-1 in einer menschlichen Probe, wobei der jeweilige Nachweis durch Detektieren eines Antikörpers gegen das jeweilige Bornavirus-spezifische Polypeptid in der Probe erfolgt, einen Träger umfassend ein Mittel zum Einfangen eines Antikörpers gegen wenigstens ein Polypeptid, das aus der Gruppe ausgewählt ist, die BoDV-1-N, BoDV-1-P, VSBV-N und VSBV-P umfasst, wobei der Träger weiter ein Mittel umfasst zum Einfangen eines Antikörpers gegen ein neurologisches Antigen aus der Gruppe umfassend NMDAR, AMPAR, GAD65, Amphiphysin, GABA(A), GABA(B), DPPX, Lgl1, CASPR2, IGLON5, SNARE, NBC1, Flotillin und ITPR, bevorzugt NMDAR; einen Kit umfassend den erfindungsgemäßen Träger sowie ein Mittel zum Nachweis eines eingefangenen Antikörpers oder eine Kontrolle zum Nachweis der Anwesenheit einer Probe; sowie die Verwendung eines Trägers umfassend ein Mittel zum Einfangen eines Antikörpers gegen wenigstens ein Polypeptid, das aus der Gruppe ausgewählt ist, die BoDV-1-N, BoDV-1-P, BoDV-1-X, VSBV-N, VSBV-P und VSBV-X umfasst, oder eines zum Nachweis eines Bornavirus geeigneten Primers oder einer ebensolchen Sonde zum Prognostizieren von post-Transplantations-Komplikationen bei einem Menschen, zur Überwachung einer Therapie einer Encephalitis oder von post-Transplantations-Komplikationen bei einem Menschen oder zur Differenzierung einer Autoimmun- und einer infektionsbedingten Encephalitis bei einem Menschen.

Bei einer Encephalitis handelt es sich um eine Entzündung des Gehirns, die durch eine Infektion, eine paraneoplastische Erkrankung bzw. durch eine Autoimmunerkrankung bedingt ist. Die Patienten leiden unter einer Vielzahl unspezifischer Symptome wie Fieber, Kopfschmerzen, Lähmungen, Sehstörungen, Krämpfen, Bewusstlosigkeit, Wahrnehmungs- und Orientierungsstörungen.

Bei rechtzeitiger Erkennung gibt es für viele Encephalitiden wirksame Behandlungsansätze, die sich in Abhängigkeit von der Ursache der Krankheit jedoch erheblich unterscheiden. Während eine durch Bakterien verursachte Gehirnentzündung mit Antibiotika behandelt werden kann, eine virale Encephalitis durch intravenöse Gabe antiviraler Mittel wie Acyclovir, so ist bei einer durch Autoimmunität bedingten Encephalitis die Verabreichung immunsuppressiver Mittel indiziert.

Bei leichter Ausprägung wird eine Encephalitis häufig gar nicht bemerkt. Je früher die Krankheit behandelt wird, desto besser, daher ist es von besonderer Bedeutung, die Diagnose trotz der unspezifischen und möglicherweise milden Symptome frühzeitig und zuverlässig stellen zu können. Falsch oder unbehandelt kann sie zu einer schweren Erkrankung mit bleibenden Schäden wie Lähmungen, Sprachstörungen und geistiger Behinderung führen. Bei bakteriellen Encephalitiden beträgt die Sterblichkeit bis zu 50 Prozent; bei einer durch Herpes simplex verursachten Encephalitis sogar 70 Prozent.

In den letzten Jahren wurde eine Vielzahl von Ausprägungen viraler oder durch Autoimmunität bedingter Encephalitiden entdeckt, beispielsweise durch Autoantikörper gegen NMDAR (EP2057466 B1), GABA(A) (EP2863231 A1), GABA(B) (EP2483417 B1), DPPX (US9719993 BB), Lgl1 (US9250250 BB), IGLON5 (EP2905622 A1), SNARE (EP17001205), NBC1 (EP3026434 A1), Flotillin (EP3101424 A1) und ITPR (EP3018478 A1) bedingte Encephalitiden.

Dennoch gibt es immer noch eine große Zahl von Patienten, die unter einer Krankheit mit für Encephalitis typischen Symptomen leiden, aber nicht richtig diagnostiziert werden können (Bloch and Glaser (2007) Diagnostic approaches for patients with suspected encephalitis. Current Infectious Disease Reports (4):315-22). Das betrifft insbesondere die Gruppe der Patienten mit einer limbischen Encephalitis, die in der Literatur als durchweg durch Autoimmunität bedingt dargestellt wird (Melzer et al. (2015) Limbic Encephalitis: Potential Impact of Adaptive Autoimmune Inflammation on Neuronal Circuits of the Amygdala. Front. Neurol. 3;6:171).

Eine besonders gefährdete Gruppe sind Patienten, die sich einer Organtransplantation unterziehen. Abgesehen von dem ohnehin geschwächten Zustand dieser Patienten begünstigt die langjährige Gabe von Medikamenten, nach der Transplantation Immunsuppressiva in hohen Dosen, den Ausbruch verschiedenster Krankheiten wie opportunistischen Infektionen. Ein bedeutendes Problem insbesondere bei der Transplantation von soliden Organen besteht in neurologischen Komplikationen, die immerhin 10% bis 59% der Empfänger betreffen (Senzolo et al. (2009) Neurologic complications after solid organ transplantation. Transpl. Int. (3):269-78, 10-59%).

In vielen Fällen ist nicht klar, wodurch diese neurologischen Komplikationen bedingt sind. In Frage kommt neben neurologischen Infektionssymptomen auch die Neurotoxizität der verabreichten Immunsuppressiva. So führt die Gabe von Corticosteroiden zu einem erhöhten Risiko einer Myopathie. Auch Leberversagen kann sich in Form neurologischer Symptome manifestieren (Shalimar et al. (2015) Management in Acute Liver Failure. J. Clin. Exp. Hepatol. (5):S104-S115).

Bornavirus-Infektionen bei Säugetieren wurden schon vor mehr als 100 Jahren beschrieben. Bei Pferden ist das Säugetier-Bornavirus BoDV-1 als Ursache für Encephalitis bekannt. Es kommt dabei zu einer andauernden Erkrankung im zentralen Nervensystem, in deren Folge sich eine nicht-eitrige Meningoencephalitis (Gehirn- und Gehirnhautentzündung) entwickelt. Erkrankte Tiere entwickeln motorische Störungen, zeichnen sich durch Verhaltensauffälligkeiten aus und sterben häufig infolge der Infektion.

Hoffmann et al. (2015) A variegated squirrel bornavirus associated with fatal human encephalitis, N Engl J Med 2015; 373, Seiten 154-162, beschreiben eine tödlich verlaufende Virusinfektion mehrerer älterer männlicher Patienten mit Vorerkrankungen (Bluthochdruck, Diabetes und/oder Adipositas) mit einem Bornavirus, das Bunthörnchen befällt ("VSBV-1", d.h. Variegated Squirrel Bornavirus-1). Sämtliche Patienten litten unter einer Thrombose, die zu pulmonalem Embolismus führte. Die Patienten litten unter Encephalitis, bei der es sich jedoch nicht um eine limbische Encephalitis handelte, sondern um ein Krankheitsbild mit ödematösen Läsionen in den cerebralen cortikalen Bereichen ohne limbische Verteilung. Mittels indirektem Immunfluoreszenztest (IIFT) basierend auf einer mit dem vollständigen BoDV-1 Virus infizierten felinen Zelllinie wurden Antikörper gegen Bornaviren nachgewiesen.

Eine Arbeitsgruppe berichtet Anfang der 2000er Jahre von einem angeblichen Zusammenhang zwischen psychiatrischen Erkrankungen und einer Infektion mit Bornaviren von der Art, die Pferde befällt. Diese Ergebnisse konnten von unabhängigen Experten nicht reproduziert werden und sind in der Fachwelt daher nicht anerkannt. Vielmehr kommt das Robert-Koch-Institut zu dem Ergebnis: "Der von Bode *et al.* (2001) beschriebene Test muss nach den vorliegenden Erkenntnissen als ungeeignet für eine aussagefähige Diagnostik angesehen werden." (https://www.rki.de/DE/Content/Forsch/Forschungsschwerpunkte/NeueRisiken/NeuartigeErrege r/Einstellung_Projekt_Bornavirus.html, abgerufen am 14. Dezember 2017). Eine durch BoDV-1 verursachte Encephalitis bei Menschen wurde bisher nicht im Stand der Technik beschrieben.

In einer Presseerklärung berichtet das Friedrich-Löffler-Institut von der Entwicklung eines Tests zum Nachweis von VSBV-1 (http://www.wir-sind-tierarzt.de/2015/07/bestaetigt-bunthoernchenuebertragen-bornaviren/ abgerufen am 14. Dezember 2017).

In WO 98/30238 wird ein Zusammenhang von Bornaviren mit neuropsychiatrischen Störungen berichtet.

WO 99/34216 offenbart, dass der Zusammenhang zwischen dem Bornavirus und einer Encephalitis aus dem Tierreich bekannt ist.

In JP H1 1180998 wird ebenfalls der Zusammenhang von Encephalomyelitis und Bornaviren bei Tieren beschrieben.

WO 98/18457 offenbart die Verwendung von Adamantanaminen oder Struktur-analoger Verbindungen zur Behandlung und Prophylaxe verschiedener Krankheitszustände (u.a. Störungen des limbischen Systems), die mit Borna-Virusinfektionen in Verbindung gebracht werden.

US 2013/0072582 offenbart ein Verfahren zur Diagnose von limbischer Enzephalitis basierend auf dem Nachweis von Antikörpern gegen LGI1 in Körperflüssigkeiten.

US 2009/155261 offenbart ein Verfahren zur Diagnose von limbischer Enzephalitis basierend auf dem Nachweis von Antikörpern gegen den NMDA-Rezeptor in Körperflüssigkeiten.

Li et al. (2009) Detection and analysis of Borna disease virus in Chinese patients with neurological disorders; European Journal ol Neurology 2009, 16: 399-403 beschreiben ein Verfahren zum Nachweis des Borna-Virus in Säugetier-Proben basierend auf dem Nachweis von viralen Nukleinsäuren. Die Ergebnisse sind jedoch aus den gleichen Gründen (wie oben beschrieben) in der Fachwelt nicht anerkannt.

Chong et al. (2001) Treatment of Acute Nipah Encephalitis with Ribavirin; Ann Neurol 2001; 49: 810-813 beschreiben die Verwendung von Ribavirin als anti-virales Medikament zur Behandlung von viraler Enzephalitis.

Vor diesem Hintergrund liegt der vorliegenden Erfindung die Aufgabe zu Grunde, ein verbessertes Nachweissystem zur Diagnose einer viralen Infektion, insbesondere einer bornaviralen Infektion bereitzustellen, bevorzugt mit verbesserter diagnostischer Zuverlässigkeit, besonders mit Hinblick auf Spezifität und/oder Sensitivität, relativ zu den im Stand der Technik beschriebenen Systemen.

Weiterhin liegt der Erfindung die Aufgabe zu Grunde, ein Nachweissystem zur Diagnose, insbesondere Differenzialdiagnose von Encephalitis, mit Schwerpunkt auf limbischer Encephalitis bereitzustellen, das insbesondere die Unterscheidung einer Autoimmun- und einer infektionsbedingten, besonders durch eine virale Infektion bedingten Encephalitis, noch bevorzugter limbischen Encephalitis gestattet.

Weiterhin liegt der Erfindung die Aufgabe zu Grunde, ein Nachweissystem bereitzustellen, das die Diagnose, Behandlung und Prävention von post-Transplantations-Komplikationen gestattet, besonders Komplikationen mit neurologischen Symptomen.

Die der Erfindung zu Grunde liegende Aufgabe wird durch den Gegenstand der beigefügten unabhängigen und abhängigen Ansprüche gelöst.

In einem ersten Aspekt wird die der Erfindung zu Grunde liegende Aufgabe gelöst durch ein Verfahren zur Diagnose einer limbischen Encephalitis, PNS, Encephaloymyelitis, Leukoencephalopathie, Retinitis oder Optikusatrophie umfassend den Schritt Nachweisen eines Mammalian 2 Bornavirus VSBV-1, und umfassend den Schritt Nachweisen eines Mammalian 1 Bornavirus BoDV-1 in einer menschlichen Probe, wobei der jeweilige Nachweis durch Detektieren eines Antikörpers gegen das jeweilige Bornavirus-spezifische Polypeptid in der Probe erfolgt.

In einem zweiten Aspekt wird die der Erfindung zu Grunde liegende Aufgabe gelöst durch ein Verfahren zum Prognostizieren von neurologischen post-Transplantations-Komplikationen oder zum Screenen von Organspendern oder Spenderorganen, umfassend Schritt Nachweisen eines Bornavirus in einer menschlichen Probe.

In einem dritten Aspekt wird die der Erfindung zu Grunde liegende Aufgabe gelöst durch ein Verfahren zur Differenzierung einer Autoimmun- und einer infektionsbedingten Encephalitis, umfassend den Schritt Nachweisen eines Bornavirus in einer menschlichen Probe.

In einer bevorzugten Ausführungsform erfolgt der Nachweis durch Detektieren eines Antikörpers gegen ein Bornavirus-spezifisches Polypeptid in der Probe, bevorzugt aus der Gruppe umfassend ein N-, P- und X-Protein des Bornavirus.

In einer weiteren bevorzugten Ausführungsform wird die Probe mit einem Träger kontaktiert, der ein Mittel zum Einfangen eines Antikörpers gegen wenigstens ein Polypeptid umfasst, das aus der Gruppe ausgewählt ist, die BoDV-1-N, BoDV-1-P, BoDV-1-X, VSBV-N, VSBV-P und VSBV-X umfasst.

In einer weiteren bevorzugten Ausführungsform wird ein eingefangener Antikörper mittels Enzymaktivität, Fluoreszenz oder Chemilumineszenz nachgewiesen.

In einer weiteren bevorzugten Ausführungsform ist der Träger ausgewählt aus der Gruppe umfassend einen membranbasierten Immunblot, bevorzugt ein Western-Blot oder ein Line-Blot, ein oder mehr als ein Bead, einen für Immunfluoreszenz hergerichteten Biochip und eine Mikrotiterplatte für ELISA.

In einem vierten Aspekt wird die der Erfindung zu Grunde liegende Aufgabe gelöst durch einen Gegenstand gemäß Anspruch 7.

In einer bevorzugten Ausführungsform umfasst der Träger weiter ein Mittel zum Einfangen eines Antikörpers gegen wenigstens ein Virus aus der Gruppe umfassend Herpex simplex HSV-1, Herpex HSV-2, Humanes Herpesvirus HHV-6, Humanes Herpesvirus HHV-7, Tollwut-Virus, LCMV, West-Nil-Virus, Tick Borne Encephalitis-Virus, Usutu-Virus, Toscana-Virus, Varizella-Zoster-Virus, Epstein-Barr-Virus, Cytomegalovirus, Equine Encephalitis-Viren, Chikungunya-Virus, La Crosse-Virus, Rift-Tal-Fieber-Virus, St. Louis-Encephalitis-Virus, Influenza-A-Virus, Masernvirus, Mumpsvirus, Rötelnvirus, Hendra-Virus, Nipah-Virus, Enterovirus, California Encephalitis Virus, Powassan-Virus, Murray-Valley-Encephalitis-Virus und Japanische-Encephalitis-Virus.

Der erfindungsgemäße Träger umfasst ein Mittel zum Einfangen eines Antikörpers gegen ein neurologisches Antigen aus der Gruppe umfassend NMDAR, AMPAR, GAD65, Amphiphysin, GABA(A), GABA(B), DPPX, Lgl1, CASPR2, IGLON5, SNARE, NBC1, Flotillin und ITPR.

In einem fünften Aspekt wird die der Erfindung zu Grunde liegende Aufgabe gelöst durch ein Kit umfassend den erfindungsgemäßen Träger sowie ein Mittel zum Nachweis eines eingefangenen Antikörpers oder eine Kontrolle zum Nachweis der Anwesenheit einer Probe.

In einem sechsten Aspekt wird die der Erfindung zu Grunde liegende Aufgabe gelöst durch eine in-Vitro-Verwendung eines Trägers umfassend ein Mittel zum Einfangen eines Antikörpers gegen wenigstens ein Polypeptid, das aus der Gruppe ausgewählt ist, die BoDV-1-N, BoDV-1-P, BoDV-1-X, VSBV-N, VSBV-P und VSBV-X umfasst, und umfassend ein Mittel zum Einfangen eines Antikörpers gegen wenigstens ein anderes Polypeptid, das aus dieser Gruppe ausgewählt ist, zur Diagnose einer Encephalitis, PNS, Encephaloymyelitis, Leukoencephalopathie, Retinitis oder Optikusatrophie.

In einem siebten Aspekt wird die der Erfindung zu Grunde liegende Aufgabe gelöst durch eine *in-*Vitro-Verwendung eines Trägers umfassend ein Mittel zum Einfangen eines Antikörpers gegen wenigstens ein Polypeptid, das aus der Gruppe ausgewählt ist, die BoDV-1-N, BoDV-1-P, BoDV-1-X, VSBV-N, VSBV-P und VSBV-X umfasst, oder eines zum Nachweis eines Bornavirus geeigneten Primers oder einer ebensolchen Sonde zum Prognostizieren von post-Transplantations-Komplikationen bei einem Menschen, zur Überwachung einer Therapie einer Encephalitis oder von post-Transplantations-Komplikationen bei einem Menschen oder zur Differenzierung einer Autoimmun- und einer infektionsbedingten Encephalitis bei einem Menschen.

In einem achten Aspekt wird die der Erfindung zu Grunde liegende Aufgabe gelöst durch eine *in-Vitro*-Verwendung eines erfindungsgemäßen Trägers oder Kits zur Differenzierung einer Autoimmun- und einer infektionsbedingten Encephalitis, bevorzugt einer limbischen Encephalitis.

In einem neunten Aspekt wird die der Erfindung zu Grunde liegende Aufgabe gelöst durch ein Verfahren zur Diagnose einer limbischen Encephalitis umfassend den Schritt Nachweisen eines Bornavirus, bevorzugt aus der Gruppe umfassend Mammalian 2 Bornavirus, noch bevorzugter VSBV-1, und Mammalian 1 Bornavirus, noch bevorzugter BoDV-1, in einer menschlichen Probe.

Hiermit wird die Verwendung eines antigenen Polypeptides aus der Gruppe umfassend BoDV-1-N, BoDV-1-P, VSBV-N, VSBV-P, NMDAR, AMPAR, GAD65, Amphiphysin, GABA(A), GABA(B), DPPX, Lgl1, CASPR2, IGLON5, SNARE, NBC1, Flotillin und ITPR oder einer Variante davon zur Herstellung eines Diagnostikums oder eine Kits zur Diagnose von Encephalitis, bevorzugt Differentialdiagnose von limbischer Encephalitis, offenbart.

Weiter wird eine Verwendung von einem gegen Bornaviren wirksamen Medikament zur Prävention oder Behandlung von post-Transplantations-Komplikationen oder von Encephalitis, bevorzugt limbischer Encephalitis offenbart.

Die vorliegende Erfindung basiert auf der überraschenden Erkenntnis der Erfinder, dass eine Infektion mit Bornaviren in Patienten, besonders menschlichen Patienten, besonders zuverlässig diagnostiziert werden kann, wenn eine entsprechende Probe auf Antikörper nicht nur gegen eine Art von Bornavirus untersucht wird, sondern auf Antikörper gegen BoDV-1 und VSBV.

Die vorliegende Erfindung basiert weiterhin auf der überraschenden Erkenntnis der Erfinder, dass eine limbische Encephalitis nicht nur in Form einer Autoimmunkrankheit, sondern auch bedingt durch eine virale Infektion auftreten kann, besonders eine Infektion mit Bornaviren, und dass sie durch Nachweis eines Antikörpers gegen ein Bornavirus diagnostiziert werden kann.

Die vorliegende Erfindung basiert weiterhin auf der überraschenden Erkenntnis der Erfinder, dass post-Tranplantations-Komplikationen umfassend neurologische Symptome und Krankheiten bzw. und Encephaloymyelitis, Leukoencephalopathie, Retinitis oder Optikusatrophie durch die Übertragung von Bornaviren, besonders BoDV-1, bedingt sein können und durch den Nachweis von Antikörpern gegen Bornaviren diagnostiziert und entsprechend behandelt werden können. Weiterhin können Organspender und Spenderorgane auf ihre Qualität überprüft werden, indem in Proben von ihnen Antikörper gegen ein Bornavirus nachgewiesen werden. Ebenso kann abgeklärt werden, ob eine prophylaktische antivirale Behandlung oder Immunisierung eines Organempfängers indiziert ist.

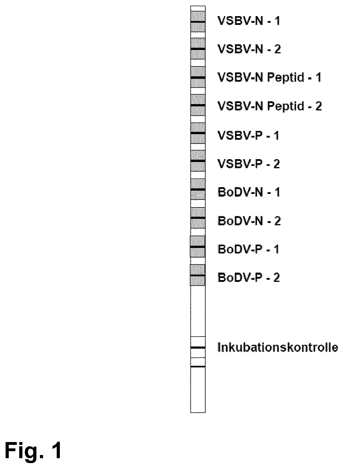

In einer bevorzugten Ausführungsform wird unter dem Begriff "limbische Encephalitis", wie hierin verwendet, eine neurologische Erkrankung des zentralen Nervensystems verstanden, bei der eine MRI-Untersuchung eine abnormale Signalintensität in den zum limbischen System gehörenden Arealen des Gehirns zeigt, besonders temporomediale Signalanhebungen, insbesondere solche, die sich nicht innerhalb weniger Tage normalisieren. Noch bevorzugter tritt wenigstens eines der drei Symptome aus der Gruppe Störung des Neugedächtnisse, Temporallappenanfälle und Affektstörung auf. Charakteristische MRI-Aufnahmen sind in der Literatur offenbart, beispielsweise in Simon/Greenberg/Aminoff, Clinical Neurology, 7th Edition, 2009, auf Seite 66 (Fig. 1-23).

In einer bevorzugten Ausführungsform wird unter dem Begriff "Diagnose", wie hierin verwendet, eine Vorgehensweise verstanden, bei der Information gewonnen wird, die die Einschätzung unterstützt oder überhaupt erst die Einschätzung gestattet, ob ein Subjekt unter einer Krankheit leidet oder mit höherer Wahrscheinlichkeit als eine durchschnittliche Person unter einer Krankheit leidet oder in der Vergangenheit litt oder in der Zukunft leiden wird, ebenso, ob eine Krankheit fortschreitet oder wie sie sich in der Zukunft entwickeln wird oder um einzuschätzen, ob eine bestimmte Behandlung anschlagen wird. Beispielsweise zeigt der Nachweis von einem Bornavirus in einer Probe von einem Spender oder einem Spenderorgan, dass die Wahrscheinlichkeit von durch ein Bornavirus bedingten post-Transplantations-Komplikationen erhöht ist. Weiterhin zeigt ein solcher Nachweis, dass ein Patient von einer antiviralen Behandlung, z.B. mit Ribavirin, profitieren kann, nicht jedoch von einer immunsuppressiven Behandlung, die darauf abzielt, die Bildung von Autoantikörpern zu verhindern oder zu verringern. In anderen Worten umfasst der Begriff "Diagnose" nicht nur das Diagnostizieren, sondern auch das Prognostizieren und das Überwachen des Krankheitsfortschrittes oder des Therapieerfolges bei einer Krankheit.

Bevorzugt ist es ausreichend für die Diagnose, lediglich nachzuweisen, ob der Antikörper vorhanden ist, wobei bestimmt werden kann, ob nachweisbare Konzentrationen des Antikörpers in der Probe vorhanden sind. In einer bevorzugten Ausführungsform wird bestimmt, ob die relative Konzentration des Antikörpers bei einem zu diagnostizierenden Patienten höher ist als bei einem durchschnittlichen Gesunden. Es kann bestimmt werden, ob die Konzentration um den Faktor 1,1, bevorzugter 1,2, 1,5, 1, 2, 5, 10, 20, 25, 50, 100, 200, 500, 1000, 10000 or 100000 höher ist als in einer Probe von einem durchschnittlichen Gesunden.

Kann ein Antikörper gegen ein Bornavirus, bevorzugt aus der Gruppe umfassend VSBV-1 oder BoDV-1, nachgewiesen werden, so spricht das für eine erhöhte Wahrscheinlichkeit, dass der Patient unter einer Bornavirus-Infektion leidet bzw. dafür, dass eine Encephalitis, bevorzugt limbische Encephalitis, durch eine virale Infektion bedingt ist, nicht durch eine Autoimmunkrankheit. Weiterhin zeigt der Nachweis eines solchen Antikörpers bei einem Patienten, der unter Encephalitis leidet, an, dass dieser Patient nicht unter einem PNS und damit unter einem mit PNS assoziierten Krebs leidet, beispielsweise einem kleinzelligen Krebs der Lunge.

Der Nachweis eines Antikörpers gegen ein Bornavirus und die daraus zu schließende Infektion mit einem Bornavirus spricht für eine therapeutische oder vorsorgliche Behandlung mit einem antiviralen Medikament oder entsprechenden therapeutischen Maßnahmen. Beispielsweise konnte gezeigt werden, dass Ribavirin gegen Bornaviren wirksam ist. Hingegen wäre die Gabe von Immunsuppressiva völlig verfehlt, da diese nicht nur mit erheblichen Nebenwirkungen verbunden sind, sondern die Abwehrkräfte des Patienten gegen virale und opportunistische Infektionen schwächen. Die Abwesenheit eines Antikörpers gegen ein Bornavirus und/oder die Anwesenheit eines Autoantikörpers, bevorzugt gegen NMDAR, spricht für eine therapeutische oder vorsorgliche Behandlung mit einem Immunsuppressivum, beispielsweise aus der Gruppe umfassend Rituximab, Prednisolon, Methylprednisolon, Cyclophosphamid, Mycophenolatemofetil, intravenöse Immunoglobuline, Tacrolimus, Cyclosporin, Methotrexat und Azathioprin.

In einer bevorzugten Ausführungsform wird differenziert, ob der zu diagnostizierende Patient einen oder mehr als einen, bevorzugt zwei oder mehr oder drei oder mehr verschiedene Antikörper gegen ein Bornavirus aufweist, wobei die Antikörper optional aus der Gruppe von Antikörpern gegen BoDV-1-N, BoDV-1-P, BoDV-1-X, VSBV-N, VSBV-P und VSBV-X, bevorzugter BoDV-1-N, BoDV-1-P, VSBV-N und VSBV-P ausgewählt sind. Insbesondere kann der Nachweis eines Antikörpers gegen ein Antigen aus dieser Gruppe anzeigen, dass der Patient unter einer frühen oder latenten Infektion leidet, wohingegen der Nachweis von Antikörpern gegen mehr als ein, bevorzugt zwei, drei oder vier Antigene aus dieser Gruppe eine klinisch apparente Infektion anzeigt.

Der Fachmann versteht, dass ein solcher Nachweis in der Regel nicht allein eine umfassende Diagnose gestattet, sondern dass weitere Aspekte berücksichtigt werden müssen, beispielsweise weitere in einer Probe zu bestimmende Parameter, die medizinische Vorgeschichte, klinische Symptome, die Anamnese oder die Ergebnisse bildgebender Verfahren, z.B. MRI. Er versteht weiter, dass der Wert des erfindungsgemäßen Verfahrens darin bestehen kann, dass eine indirekte Diagnose oder Differentialdiagnose erfolgen kann, bei der der Ausschluss einer Krankheit anzeigt, dass der Patient unter einer anderen Krankheit mit ähnlichen Symptomen leidet. In einer bevorzugten Ausführungsform kann der Nachweis von einem Antikörper gegen ein Bornavirus anzeigen, dass der Patient unter einer limbischen Encephalitis leidet, die nicht eine Autoimmunkrankheit ist. Weiterhin kann solch ein Nachweis anzeigen, dass der Patient nicht unter einem paraneoplastischen Syndrom und/oder einem Krebs leidet, bevorzugt einem kleinzelligen Lungenkrebs. Umgekehrt kann der Nachweis von einem Autoantikörper gegen den NMDA-Rezeptor anzeigen, dass der Patient nicht unter einer viral bedingten Encephalitis, besonders limbischen Encephalitis leidet.

In einer bevorzugten Ausführungsform misst der Fachmann hierin genannten klinischen Symptomen die Bedeutung zu, wie sie zum Zeitpunkt des Prioritätstages aus entsprechenden Lehrbüchern hervorgeht, beispielsweise Simon/Greenberg/Aminoff, Clinical Neurology, 7th Edition, 2009.

Das Bornavirus ist bevorzugt ein Bornavirus, das Säugetiere befällt. Noch bevorzugter ist das Bornavirus ein Bornavirus, das Menschen befällt. In einer besonders bevorzugten Ausführungsform ist das Bornavirus aus der Gruppe ausgewählt, die Mammalian 2 Bornavirus, noch bevorzugter VSBV-1, und Mammalian 1 Bornavirus, noch bevorzugter BoDV-1, umfasst.

In einer bevorzugten Ausführungsform umfasst "Mammalian 1 Bornavirus", wie hierin verwendet, AJ311524_BoDV-2_1998, AJ311522_BoDV-1_1980, BDU04608_BoDV-1_1929, AJ311523_BoDV-1_1994, AB246670_BoDV-1_2004, und AB258389_BoDV-1_1997, vergleiche Tappe et al. (2018) Fatal limbic encephalitis in a zoo animal caretaker caused by variegated squirrel bornavirus: Zoonotic bornaviruses as occupational risk, Manuskript eingereicht.

In einer bevorzugten Ausführungsform umfasst "Mammalian 2 Bornavirus", wie hierin verwendet, LT594387_BH122/15-2_*C*. *prevostii*_2015, KY488724_BH22/16-36_*C.prevostii*_2016, KY488723_BH22/16-37_*C*. *prevostii_*2016, KY488727_BH22/16-16_*T*. *swinhoei_*2016, LT594389_BH49/15-1_*C*. *prevostii*_2015, KY488726_BH22/16-34_*C.prevostii*_2016, LT594388_BH133/15_*C*. *prevostii*_2015, KY488722_BH22/16-38_*C*. *prevostii_*2016, KY488725_BH22/16-35_*C*. *finlaysonii*_2016, LT594383_BH3/16-2_*C*. *prevostii_*2016, LT594381_BH122/15-1_*C*. *prevostii*_2015, LT594382_BH48/15-1_*S. variegatoides*_2015*,* LN713681_*H*. *sapiens*_2015, LN713680_*S*. *variegatoides*_2015, LT594386_BH3/16-1_*C*. *prevostii_*2016, LT594384_BH49/15-6_*S*. *variegatoides*_2015, LT594385_BH49/15-11_*S*. *variegatoides*_2015*,* LT594391_BH48/15-2_S. variegatoides_2015, LT594390_BH75/15_*S*. *variegatoides*_2015, KY488728_BH21/16_*C.prevostii*_2016, MF597762_BH55/16_*H*. *sapiens*_2016 und KY488729_BH12/16_*C. prevostii_*2016, vergleiche Tappe *et al.* (2018) Fatal limbic encephalitis in a zoo animal caretaker caused by variegated squirrel bornavirus: Zoonotic bornaviruses as occupational risk, Manuskript eingereicht.

Sämtliche Bornaviren können durch Vollgenomsequenzierung unter Verwendung der Methodologie identifiziert werden, wie sie in Shahhosseini N, Lühken R, Jöst H, Jansen S, BörstlerJ, Rieger T, Krüger A, Yadouleton A, de Mendonça Campos R, Cirne-Santos CC, Ferreira DF, Garms R, Becker N, Tannich E, Cadar D, Schmidt-Chanasit J. (2017) Infect Genet Evol.; 55:260-268 beschrieben ist, in Kombination mit den von Tappe et al. (2018) Fatal limbic encephalitis in a zoo animal caretaker caused by variegated squirrel bornavirus: Zoonotic bornaviruses as occupational risk, Manuskript eingereicht, publizierten Daten.

Die zu untersuchende Probe ist für den Fall, dass Antikörper nachgewiesen werden, eine Probe, die ein repräsentatives Set von Antikörpern des Patienten enthält. Bevorzugt ist sie aus der Gruppe ausgewählt, die Serum, Plasma, Speichel und CSF umfasst.

In einer besonders bevorzugten Ausführungsform wird das Virus auch molekulargenetisch unter Verwendung Bornavirus-spezifischer Primer und Sonden identifiziert und nachgewiesen, bevorzugt über PCR, noch bevorzugter Reverse Transkriptase-PCR. Die Primer und/oder Sonden können für BoDV-1 und/oder für VSBV-1 spezifisch sein. Beispielhafte Primer, Sonden und ein entsprechendes Protokoll für den Nachweis von VSBV-1 sind in Hoffmann et al. (2015) A variegated squirrel bornavirus associated with fatal human encephalitis, N Engl J Med 2015; 373, Seiten 154-162 beschrieben. Entsprechende Reagenzien und Verfahren für einen Breitband-Test zum Nachweis aller bekannten Säugetier-Bornaviren und der meisten Vögel befallenden Bornaviren sowie Reagenzien und Verfahren zum Nachweis von BoDV-1 sind in Bourg et al. (2016) Virology Journal; 13:151, beschrieben.

Kurz gesagt kann ein Bornavirus in eine menschlicher Probe molekulargenetisch dadurch nachgewiesen werden, dass zunächst virale Nukleinsäuren aus der Probe unter Verwendung handelsüblicher Kits extrahiert wird [MagMAX^{™} Viral RNA Isolation Kit (Life Technologies, Carlsbad, California, USA)]. Anschließend kann das Virusgenom durch Sequenzierung [Schlottau et al., 2018] und Vergleich der erhaltenen Sequenz mit bekannten Sequenzen aus der Literatur bestimmt werden, beispielsweise [Schlottau et al., 2018; GenBank: LN713681 für VSBV-1; KU748787, AJ311521, AY374521, AY374519, für BoDV-1; AJ311524 für BoDV-2].

Alternativ kann ein Bornavirus mittels RT-PCR nachgewiesen werden. Dazu kann virale RNA unter Verwendung geeigneter Kits wie dem Viral Mini Kit (Qiagen) extrahiert werden. Der Nachweis eines Bornavirus kann durch Amplifizieren eines 61 bp-Fragmentes des P-Genes und des Endes des X-Gens unter Verwendung der in Bourg et al. (2016) beschriebenen Primer "forward primer Borna-2048-F": 5'-CGC GAC CMT CGA GYC TRG T-3' und "reverse primer Borna-2118-R": 5'-GAC ARC TGY TCC CTT CCK GT-3' erfolgen. Die Detektion erfolgt durch Agarose-Gelelektrophorese. Ein quantitativer Nachweis von BODV-1 kann durch Amplifikation eines 161 bp-Fragmentes des X- und P-Gens unter Verwendung folgender Primer und Sonde nachgewiesen werden: BoDV1-1288-F TAGTYAGGAGGCTCAATGGCA, BoDV-1449-R GTCCYTCAGGAGCTGGTC, BoDV1-1346-Sonde FAM-AAGAAGATCCCCAGACACTACGACG-BHQ1 (Schlottau et al., 2018). Ein quantitativer Nachweis von BODV-1 kann durch Amplifikation eines 74 bp-Fragmentes des M- und G-Gens unter Verwendung folgender Primer und Sonde nachgewiesen werden: BoDV1-2262-F CAATYAATGCAGCYTTCAATGTCTT, BoDV1-2336-R GAATGTCYGGGCCGAGAG, BoDV-2316-Sonde FAM-CCARCACCAATGTTCCGAAGCCG-BHQ1 (Schlottau et al., 2018).

Das Virus kann auch immunhistochemisch durch Untersuchung von Gewebeschnitten nachgewiesen werden. Dies ist bei der Untersuchung von Spenderorganen, zu denen keine Blutproben vorliegen, eine Option.

In einer bevorzugten Ausführungsform wird unter dem Begriff "post-Transplantations-Komplikationen", wie hierin verwendet, Komplikationen nach einer Transplantation eines Körperteils verstanden, bevorzugt eines Gewebes oder festen Organs, noch bevorzugter eines festen Organs, das noch bevorzugter aus der Gruppe ausgewählt ist, die Leber, Niere, Lunge, Darm, Pankreas und Herz umfasst. In einer besonders bevorzugten Ausführungsform umfassen die Komplikationen das Auftreten neurologischer Symptome und können eine Encephalitis, bevorzugt limbische Encephalitis umfassen. In einer weiteren besonders bevorzugten Ausführungsform treten bei "post-Transplantations-Komplikationen" Infektionen auf. In einer bevorzugten Ausführungsform ist ein neurologisches Symptom aus der Gruppe ausgewählt, die Tetraplegie, Neuropathie, Bewusstseinstrübung, Dysarthrie, Bradykinesie, Tremor, unsicherer Gang, beeinträchtigte kognitive Fähigkeiten und Erinnerung und Neuritis umfasst. In einer besonders bevorzugten Ausführungsform umfassen die post-Transplantations-Komplikation eine eine Beeinträchtigung der Sehfunktion, bevorzugt neurologische ophthalmologische Symptome und/oder Krankheiten, besonders bevorzugt eine Optikusatrophie.

Zeitlich handelt es sich bei "post-Transplantations-Komplikationen" bevorzugt um Komplikationen, die nach dem operativen Eingriff beim Empfänger auftreten, bevorzugt bis zu einem Monat, in den ersten sechs Monaten oder später als sechs Monate nach dem Eingriff.

Für das Screenen von Organspendern oder Spenderorganen kann den Spendern oder ihren Organen eine geeignete Probe entnommen werden, bevor die Transplantation durchgeführt wird. Bei Spendern handelt es sich bevorzugt um eine Blut-, Speichel- oder CSF-Probe. Bei Organen werden Blut- oder Gewebereste bzw. -proben untersucht. Das Gewebe kann aus einem Organ stammen, das aus der Gruppe umfassend Leber, Niere, Lunge, Darm, Pankreas, Herz und Gehirn ausgewählt ist.

In einer bevorzugten Ausführungsform wird unter einer "Autoimmun-Encephalitis", wie hierin verwendet, eine Encephalitis verstanden, die mit dem Auftreten von einem oder mehr als einem Autoantikörper assoziiert ist. Der Autoantikörper ist bevorzugt ein Autoantikörper gegen ein neuronales Autoantigen, bevorzugt ausgewählt aus der Gruppe, die NMDAR (EP2057466 B1), GABA(A) (EP2863231 A1), GABA(B) (EP2483417 B1), DPPX (US9719993 BB), Lgl1 oder CASPR2 (US9250250 BB), IGLON5 (EP2905622 A1), SNARE (EP17001205), NBC1 (EP3026434 A1), Flotillin (EP3101424 A1), DAGLA (EP18196867), SNARE (EP17001205) und ITPR (EP3018478 A1) umfasst. Geeignete Sequenzen von Autoantigenen sind in den in Klammern angegebenen Patentschriften offenbart.

In einer bevorzugten Ausführungsform wird ein Bornavirus in einer Probe nachgewiesen. Dies bedeutet, dass das Virus oder ein von ihm abgeleitetes Molekül oder ein Antikörper des Patienten gegen das Virus oder das von ihm abgeleitete Molekül in der Probe nachgewiesen wird.

Durch Autoimmunität bedingte Encephalitis, insbesondere NMDA-Rezeptor-Enzephalitis, nicht aber eine durch Infektion mit einem Bornavirus bedingte Encephalitis, ist häufig mit dem Auftreten von Tumoren assoziiert. In einer bevorzugten Ausführungsform wird unter dem Begriff "PNS" (paraneoplastisches neurologisches Syndrom), wie hierin verwendet, eine systemische Krankheit verstanden, die indirekt durch die Anwesenheit eines Tumors verursacht wird, beispielsweise dadurch, dass der Tumor Epitope produziert oder Substanzen wie Hormone freisetzt oder in erhöhter Menge freisetzt, die die Zelle, von der der Tumor abgeleitet ist, unter vergleichbaren Umständen nicht oder nicht in solchen Mengen freisetzen würde. Die direkte oder indirekte Folge kann die Bildung von Autoantikörpern sein, die bevorzugt gegen Strukturen oder Teile des Nervensystems gerichtet sind, und die in jedem Fall direkt oder indirekt mit dem Auftreten neurologischer Symptome assoziiert sind und diese bevorzugt verursachen. Es ist bekannt, dass die NMDA-Rezeptor-Encephalitis mit dem Auftreten von Teratomen der Ovarien assoziiert sein kann. Der Tumor kann bei einer durch Autoimmunität bedingten Encephalitis nicht nachweisbar sein.

Der erfindungsgemäße Nachweis kann über den Nachweis eines für das Bornavirus spezifischen Polypeptides oder eines Antikörpers dagegen, bevorzugt eines Antikörpers dagegen erfolgen. Der Antikörper ist bevorzugt ein Antikörper gegen ein virales Polypeptid aus der Gruppe umfassend BoDV-1-N, BoDV-1-P, BoDV-1-X, VSBV-N, VSBV-P und VSBV-X, bevorzugt BoDV-1-N, BoDV-1-P, VSBV-N und VSBV-P. In einer besonders bevorzugten Ausführungsform wird nachgewiesen, ob der Patient ein, zwei, drei, vier oder mehr als ein, zwei, drei, vier, fünf oder sechs Antikörper gegen ein virales Polypeptid aus der Gruppe umfassend BoDV-1-N, BoDV-1-P, BoDV-1-X, VSBV-N, VSBV-P und VSBV-X aufweist.

In einer bevorzugten Ausführungsform umfasst der Träger als Mittel zum Einfangen ein Nukleoprotein von ca. 40 kDa eines Bornavirus. In einer bevorzugten Ausführungsform umfasst der Träger als Mittel zum Einfangen ein Phosphoprotein von ca. 23 kDa eines Bornavirus. In einer bevorzugteren Ausführungsform umfasst der Träger als Mittel zum Einfangen ein oder mehr als ein, zwei, drei, vier, fünf oder sechs Polypeptide aus der Gruppe umfassend BoDV-1-N, BoDV-1-P, BoDV-1-X, VSBV-N, VSBV-P und VSBV-X, bevorzugt BoDV-1-N, BoDV-1-P, VSBV-N und VSBV-P oder Varianten davon. In einer bevorzugten Ausführungsform umfasst der Träger BoDV-1-N und BoDV-1-P oder Varianten davon. In einer bevorzugten Ausführungsform umfasst der Träger VSBV-N und VSBV-P oder Varianten davon. In einer bevorzugten Ausführungsform umfasst der Träger BoDV-1-P und VSBV-P oder Varianten davon. In einer bevorzugten Ausführungsform umfasst der Träger VSBV-N und BoDV-1-N oder Varianten davon.

In einer bevorzugten Ausführungsform werden unter "BoDV-1-N", "BoDV-1-P", "VSBV-N" und "VSBV-P" die Sequenzen verstanden, die durch die Uniprot-Einträge P0C796 (BoDV-1-N), P0C798 (BoDV-1-P), A0A0H5BWD6 (VSBV-N) bzw. A0A0H5BWK0 (VSBV-P) zum Zeitpunkt des Prioritätstages der vorliegenden Patentanmeldung dargestellt werden. VSBV-X weist bevorzugt die Sequenz ART67059.1 auf, BoDV-1-X bevorzugt die Sequenz AIK27011.1. Für sämtliche Einstellungen ist die am Prioritätstag verfügbare Version der Datenbank und der an diesem Tag abrufbare Stand der Daten maßgeblich. In einer bevorzugten Ausführungsform wird unter einem N-, P- oder X-Protein eines Bornavirus ein Protein mit wenigstens 60, 70, 80, 85, 90, 95, 98 oder 99 % Sequenzidentität zu den Sequenzen P0C796, P0C798 bzw. AIK27011.1 verstanden, besonders bevorzugt eine Variante davon.

In einer bevorzugten Ausführungsform handelt es sich bei dem erfindungsgemäß eingesetzten Träger um eine medizinische, bevorzugt diagnostische Vorrichtung, die ein Mittel zum Einfangen eines oder mehr als eines Antikörpers enthält. Unter dem Begriff "Mittel zum Einfangen eines Antikörpers", wie hierin verwendet, wird ein Agens verstanden, das an einen nachzuweisenden Antikörper spezifisch derart bindet, dass dieser Antikörper allein oder in einer ausreichenden Menge stärker als ein anderer Antikörper bindet, besonders als ein anderer Antikörper, der an ein homologes Polypeptid bindet und bevorzugt ein falsch-positives Ergebnis auslösen könnte.

Insbesondere zur Differenzialdiagnose, bei der das Ziel darin liegen kann, bei einem Patienten mit neurologischen Symptomem zu unterscheiden, ob er an einer autoimmun- oder infektionsbedingten Encephalitis, bevorzugt limbischen Encephalitis leidet, ist es vorteilhaft, Antikörper gegen NMDAR einerseits und Antikörper gegen BoDV-1-N, BoDV-1-P, VSBV-N, VSBV-P oder andere Viren oder davon abgeleitete Antigene, die bei einer Infektion ähnliche oder verwechselbare Symptome verursachen, gleichzeitig einzufangen und/oder zu detektieren. Ein dazu geeigneter Träger kann neben NMDAR und/oder wenigstens einem Antigen aus der Gruppe umfassend BoDV-1-N, BoDV-1-P, VSBV-N, VSBV-P wenigstens ein Antigen eines anderen Virus aufweisen, bevorzugt mehrere Antigene anderer Viren. Ebenfalls sinnvoll sind weitere Antigene, mit denen weitere Autoantikörper detektiert werden können. In diesem Fall ist es besonders wahrscheinlich, dass mit einem Untersuchungsdurchgang ein positiver Nachweis mit einem Antigen erzielt werden kann und nicht mit einer Ausschluss-basierten Diagnose gearbeitet werden muss.

In einer besonders bevorzugten Ausführungsform bedeutet der Begriff "spezifisch binden", wie hierin verwendet, bei einem Antikörper, dass er gegen das entsprechende Antigen in einer Bindungsreaktion bindet, die durch eine Dissoziationskonstante gekennzeichnet ist, die 1 × 10⁻⁵ M, bevorzugter 1 × 10⁻⁷ M, bevorzugter 1 × 10⁻⁸ M, bevorzugter 1 × 10⁻⁹ M, bevorzugter 1 × 10⁻¹⁰ M, bevorzugter 1 × 10⁻¹¹ M, bevorzugter 1 × 10⁻¹² M oder eine stärkere Bindungsreaktion bindet. Bevorzugt wird die Dissoziationskonstante dabei durch Surface Plasmon Resonanz unter Verwendung eines Biacore-Gerätes bei 25 °C und in PBS-Puffer bei pH 7 gemessen und unter Verwendung der vom Hersteller angebotenen Software berechnet.

In einer besonders bevorzugten Ausführungsform handelt es sich bei dem Mittel zum Einfangen eines Antikörpers um ein Polypeptid, das Epitope aufweist, die von dem nachzuweisenden Antikörper erkannt werden, oder Varianten davon. Alternativ können auch Peptidomimetika solcher Polypeptide mit entsprechender Bindungsfähigkeit gegen den nachzuweisenden Antikörper eingesetzt werden, beispielsweise Peptoide, beta-Peptide und Peptide mit unnatürlichen Aminosäuren. Der Fachmann ist mit derartigen Molekülen und ihrem Design vertraut (Pelay Gimeno M, Glas A, Koch O, Grossmann TN (2015). "Structure-based design of inhibitors of protein-protein interactions: Mimicking peptide binding epitopes". Angewandte Chemie International Edition. 54 (31): 8896-8927). Beispielhafte Polypeptide als Mittel zum Einfangen umfassen SEQ ID NO7 zum Einfangen von Antikörpern gegen die NR1-Untereinheit des menschlichen NMDA-Rezeptors, deren Dimere oder Komplexe davon mit anderen Untereinheiten des NMDA-Rezeptors wie NR2A (SEQ ID NO3 aus der EP2057466) oder NR2B (SEQ ID NO1 aus der EP2057466) oder NR2C (SEQ ID NO5 aus der EP2057466 B1) zum Einfangen von Antikörpern gegen den NMDA-Rezeptor, SEQ ID NO1 und/oder SEQ ID NO2 aus der EP2863231 A1 zum Einfangen von Antikörpern gegen menschlichen GABA(A)-Rezeptor, SEQ ID NO1, SEQ ID NO 2 oder SEQ ID NO3 aus der EP2483417 B1 zum Einfangen von Antikörpern gegen menschlichen GABA(B)-Rezeptor, SEQ ID NO1, SEQ ID NO2, SEQ ID NO3, oder SEQ ID NO4 aus der WO2014/041035A1 zum Einfangen von Antikörpern gegen DPPX, SEQ ID NO2 aus der US9250250 BB zum Einfangen von Antikörpern gegen Lgl1 oder CASPR2 (US9250250 BB), zum Einfangen von Antikörpern gegen IGLON5 SEQ ID NO1 aus der EP2905622 A1, zum Einfangen von Antikörpern gegen NSF SEQ ID NO 1 aus der EP17001205, zum Einfangen von Antikörpern gegen STX1V SEQ ID NO 3 aus der EP17001205, zum Einfangen von Antikörpern gegen VAMP2 SEQ ID NO 6 aus der EP17001205 zum Einfangen von Antikörpern gegen NBC1 die Sequenz Q9Y6R1 aus Uniprot (vergleiche EP3026434 A1), zum Einfangen von Antikörpern gegen Flotillin O75955 (Flotillin1, Uniprot) und/oder Q14254 (Flotillin2, Uniprot) (vergleiche EP3101424 A1) und zum Einfangen von Antikörpern gegen ITPR Q14643 (Uniprot) (vergleiche EP3018478 A1) und jeweils Varianten davon. Bei dem Mittel zum Einfangen kann es sich auf um das Virus selbst handeln, bevorzugt in Form einer Zelle, bevorzugter einer Säugetierzelle, die mit dem Virus infiziert ist. Die Zelle ist bevorzugt isoliert und/oder aufgereinigt.

Die erfindungsgemäße Lehre kann nicht nur unter Verwendung der Wildtypsequenzen der angegebenen Polypeptide oder der Referenzsequenzen, wie sie in Form von SEQ ID NOs, zusammen von nun an als "Volllängen-Polypeptid" bezeichnet, oder Datenbankencodes oder in sonstiger Weise, ggf. implizit, hier angegeben sind, ausgeführt werden, sondern auch unter Verwendung von Varianten dieser Sequenzen.

In einer besonders bevorzugten Ausführungsform bedeutet der Begriff "Variante", wie hierin verwendet, dabei wenigstens ein Fragment des Volllängen-Polypeptids, das am C- und/oder N-terminalen Ende gegenüber dem Volllängen-Polypeptid um eine oder mehr als eine Aminosäure verkürzt ist. Ein solche Fragment kann eine Länge von 5, 6, 7, 8, 10, 12, 15, 20, 25, 50, 75, 100, 150, 200, 250, 300, 400, 500 oder 600 aufeinanderfolgenden Aminosäuren aus der Sequenz des Volllängen-Polypeptides enthalten, bevorzugt 10 oder mehr.

In einer weiteren bevorzugten Ausführungsform umfasst der Begriff "Variante", wie hierin verwendet, nicht nur ein Fragment, sondern auch ein Polypeptid, das Aminosäuresequenzen mit in der Reihenfolge zunehmender Bevorzugung wenigstens 40, 50, 60, 70, 75, 80, 85, 90, 92, 94, 95, 96, 97, 98 oder 99 % Sequenzidentität zum Volllängen-Polypeptid oder zum Fragment davon aufweist, wobei keine für die biologische Aktivität, z.B. die Fähigkeit eines Antigens, an einen für das Volllängen-Polypeptid-Antigen spezifischen Antikörper zu binden, essentielle Aminosäure deletiert oder nicht-konservativ substituiert wird. Der Stand der Technik offenbart Verfahren zur Feststellung der Sequenzidentität von Aminosäuresequenzen, z.B. in Arthur Lesk (2008), Introduction to bioinformatics, Oxford University Press, 2008, dritte Ausgabe. In einer bevorzugten Ausführungsform wird die ClustalW-Software Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) unter Verwendung der Standardeinstellungen dazu benutzt.

Zusätzlich kann ein erfindungsgemäß verwendetes Polypeptid oder eine Variante davon chemische Modifikationen aufweisen, z.B. Isotopenlabel, kovalente Modifikationen wie Glykosylierung, Phosphorylierung, Acetylierung, Decarboxylierung, Citrullinierung, Methylierung, Hydroxylierung usw.

Varianten können auch Fusionsproteine mit anderen bekannten Polypeptiden oder Varianten davon sein, bevorzugt mit einem Tag zur Aufreinigung, noch bevorzugter aus der Gruppe umfassend His Tag, Thioredoxin, Maltose-bindendes Protein, Streptavidin, Glutathion-S-Transferase oder einer Variante davon, und aktive Teile oder Domänen sein oder aufweisen, bevorzugt mit einer Sequenzidentität von wenigstens 70, 75, 80, 85, 90, 92, 94, 95, 96, 97, 98 oder 99 % zum Volllängen-Polypeptid aufweisen. In einer bevorzugten Ausführungsform wird unter dem Begriff "aktive Teile oder Domänen" ein Fragment des Volllängen-Polypeptids oder eine Variante verstanden, das bzw. die wenigstens einen Teil der biologischen Aktivität des Volllängen-Polypeptids behält.

Es ist essentiell, dass die Variante des Volllängen-Polypeptids biologische Aktivität aufweist. In einer bevorzugten Ausführungsform handelt es sich bei der biologischen Aktivität um die Fähigkeit eines Antigens, einen Antikörper einzufangen, der spezifisch an das Volllängen-Polypeptid bindet. Eine Variante von BoDV-1-N, BoDV-1-P, BoDV-1-X, VSBV-N, VSBV-P und VSBV-X hat also die Fähigkeit, einen Antikörper gegen BoDV-1-N, BoDV-1-P, BoDV-1-X, VSBV-N, VSBV-P bzw. VSBV-X einzufangen. Bevorzugt handelt es sich um einen Antikörper, der spezifisch im Blut eines Patienten vorkommt, der an einer bestimmten Krankheit leidet. Beispielsweise hat eine Variante eines Antigens zum Einfangen eines Antikörpers gegen ein Bornavirus die Fähigkeit, spezifisch einen Antikörper aus einem Patienten gegen dieses Antigen des Bornavirus zu binden. Ob das der Fall ist, kann unter Verwendung eines Immunblots bestimmt werden, wie in Beispiel 1 beschrieben ist.

Erfindungsgemäße Varianten umfassen beispielsweise SEQ ID NO 1 (Variante von VSBV-N), SEQ ID NO 3 (VSBV-P), SEQ ID NO 4 (BoDV-1-N) und SEQ ID NO 5 (BoDV-1-P).

In einer bevorzugten Ausführungsform ist das Mittel zum Einfangen ein isoliertes und/oder aufgereinigtes Polypeptid, das besonders bevorzugt rekombinant ist. Unter einem "isolierten" Polypeptid kann dabei verstanden werden, dass das Polypeptid aus seiner natürlichen Umgebung entfernt wurde. Unter einem "aufgereinigten" Polypeptid wird in einer bevorzugten Ausführungsform ein Polypeptid verstanden, das unter Verwendung von chromatographischen Verfahren gegenüber der Konzentration und/oder Reinheit in seiner natürlichen Umgebung angereichert wurde, die bevorzugt aus der Gruppe ausgewählt sind, die Affinitätschromatographie, Gelfiltrationschromatographie und lonenaustauschchromatographie umfasst. In einer besonders bevorzugten Ausführungsform ist ein Polypeptid aufgereinigt, wenn es eine Reinheit von wenigstens 40, 50, 60, 70, 80, 90, 95, 98, 99 oder 99,5% hat, was visuell oder, bevorzugt, durch Intensitätsmessung von Banden auf einem SDS-PAGE-Gel nach Coomassie-Färbung beurteilt werden kann.

Ein Polypeptid kann in Form eines Gewebes oder einer Zelle bereitgestellt werden, bevorzugt ein rekombinantes Gewebe oder eine rekombinante Zelle. Die Zelle ist bevorzugt eine eukaryontische Zelle, bevorzugter eine Insekten-, Pflanzen- oder Säugetierzelle, noch bevorzugter eine Säugetierzelle, am bevorzugtesten eine menschliche Zelle.

Ein Polypeptid kann in gefalteter Form oder in ungefalteter Form bereitgestellt werden. Bevorzugt wird die Faltung mittels CD-Spektroskopie in einem Puffer gemessen, der die Messung gestattet und in dem das Protein seine dreidimensionale Faltung annehmen kann (vergleiche beispielsweise Banaszak L. J. (2008), Foundations of Structural Biology, Academics Press, or Teng Q. (2013), Structural Biology: Practical Applications, Springer), z.B. in 10 mM Natriumphosphat bei pH 7. Bevorzugt nimmt das Protein eine Faltung an, die von eine nachzuweisenden Antikörper erkannt wird.

Ein Mittel zum Einfangen ist immobilisiert, bevorzugt an einem Träger, noch bevorzugter über eine kovalente oder nicht-kovalente Bindung.

In einer bevorzugten Ausführungsform wird ein Antikörper nach dem Einfangen durch eine Methode aus der Gruppe nachgewiesen, die Immundiffusion, Immunoelektrophorese, Lichtstreuung, Agglutination, Radioaktivität, enzymatische Aktivität, (Elektro)-Chemolumineszenz und Immunfluoreszenz umfasst. Im Falle einer Detektion durch Radioaktivität, enzymatische Aktivität, (Elektro-)Chemolumineszenz und Immunfluoreszenz kann eine detektierbare Markierung eingesetzt werden. Sie ist bevorzugt an einem sekundären Antikörper angebracht, der an den nachzuweisenden Antikörper bindet. Nachweismethoden sind beispielsweise in in Zane, H. D. (2001), Immunology - Theoretical & Practical Concepts in Laboratory Medicine, W. B. Saunders Company, besonders in Kapitel 14 beschrieben.

In einer bevorzugten Ausführungsform ist der nachzuweisende Antikörper ein Antikörper aus der Gruppe umfassend Immunglobulin Klasse G (IgG), Immunglobulin Klasse M (IgM) und Immunglobulin Klasse A (IgA), bevorzugt G oder M, noch bevorzugter G. In einer weiteren bevorzugten Ausführungsform werden zwei oder mehr Klassen von Antikörpern aus der Gruppe umfassend Immunglobulin Klasse G, Immunglobulin Klasse M und Immunglobulin Klasse A, bevorzugt G und M, nachgewiesen.

Der nachzuweisende Antikörper kann quantifiziert werden. In einer bevorzugten Ausführungsform wird der nachzuweisende Antikörper an mehr als einem Tag nachgewiesen und bevorzugt quantifiziert. Noch bevorzugter handelt es sich um einen Antikörper der Klasse G (IgG). Auf diese Weise kann der Verlauf des Titers verfolgt werden. Dabei gestattet ein im Verlauf ansteigender IgG-Titer das Erkennen einer aktiven Infektion mit einem Bornavirus und das Unterscheiden einer aktiven Infektion mit einem Bornavirus von einer passiven Immunisierung oder einem länger zurückliegenden Kontakt bzw. einer Infektion mit dem Erreger.

In einer bevorzugten Ausführungsform ist der Träger ausgewählt aus der Gruppe umfassend einen membranbasierten Immunblot, bevorzugt ein Western-Blot, Lateral Flow Assay oder ein Line-Blot, ein oder mehr als ein Bead, einen für Immunfluoreszenz hergerichteten Biochip und eine Mikrotiterplatte für ELISA.

Erfindungsgemäß wird ein Kit bereitgestellt, der den erfindungsgemäßen Träger umfasst, zusätzlich optional eine Anleitung und/oder Puffer und Reagenzien, bevorzugt aus der Gruppe umfassend eine Waschlösung, eine Lösung umfassend ein Mittel zum Nachweis eines eingefangenen Antikörpers und eine Lösung umfassend eine Positivkontrolle. Statt einer Lösung kann das jeweilige Reagenz auch in trockener Form, beispielsweise als Pulver, bereitgestellt werden, bevorzugt mit einer Lösung, in der es bei Bedarf gelöst werden kann. Als Positivkontrolle kann ein nachzuweisender Antikörper oder eine Variante davon dienen, die spezifisch an das Antigen bindet, an das der Antikörper bindet. Bevorzugt umfasst der Träger eine Kontrolle zum Nachweis der Anwesenheit einer Probe, bevorzugt einer Serumprobe. Beispielsweise kann ein in jeder Serumprobe vorhandenes Protein nachgewiesen werden, um die Anwesenheit einer Serumprobe zu bestätigen. Als Mittel zum Nachweis eines eingefangenen Antikörpers dient bevorzugt ein sekundärer Antikörper mit einem detektierbaren Label.

In der vorliegenden Patentanmeldung sind neue Polypeptide und/oder Nukleinsäuren aufgeführt. Sie haben die folgenden Sequenzen:
**SEQ ID NO 1 (VSBV-N (40 kDa, His-Tag))** 
MNITMPPKRRLLEDPDVMDDQEPEPTSPPMPKLPGKFLQYTVGGSDPHPGIGEEKDIKHNAVA
LLDSSRRDMFHPVTPSLVFLCLLIPGLHAAFLHGGVPKESYLSTPISRGEQTFVKVSRFYGERTA
SRELTELEISSIFNHCCSLLIGVVIGSSAKIRAGAEQIKKRFKTLMASLNRPSHGETATLLQMFNP
HEAIDWINGQPWVGSLVLSLLTTDFESPGKEFMDQIKLVASYAQMTTYTTIKEYLAECMDATLTI
PAVAHEIREFLEISAKLKNEHAELFPFLGAIRHPDAIKLAPRSFPNLASAAFYWSKKENPTMAGY
RASTIQPGATVKETQLARYRRREVSRGEDGAELSGEISDIMKMIGVTGLVLEHHHHHH
**SEQ ID NO 2 VSBV-N Peptid (30 kDa, His-GST-Tag))** 
MSHHHHHHHHMSPILGYWKIKGLVQPTRLLLEYLEEKYEEHLYERDEGDKWRNKKFELGLEFP
NLPYYIDGDVKLTQSMAIIRYIADKHNMLGGCPKERAEISMLEGAVLDIRYGVSRIAYSKDFETLK
VDFLSKLPEMLKMFEDRLCHKTYLNGDHVTHPDFMLYDALDVVLYMDPMCLDAFPKLVCFKKR
IEAIPQIDKYLKSSKYIAWPLQGWQATFGGGDHPPKLEVLFQGPAMFVKVSRFYGERTASR
**SEQ ID NO 3 (VSBV-P (23 kDa, His-Tag))** 
MASRPSSLVESLEDEESLQTPRRVRSRSPRPKRIPQDALTQPVDRLLKNIKKNPSMISDPEQRT
GREQLSNDELIKQLVTELAENSMIEAEGLRGALDDISSKVDSGLESISSLQVETLQTVQKTDYAD
SIKTLGENIKVLDRSMKTMMETMRLMMEKIDLLYASTAIGQSNTPMLPSHPAQPRLYPTLPSAP
TADEWDILPLEHHHHHH
**SEQ ID NO 4 (BoDV-1-N (40 kDa, His-Tag))** 
MNITMPPKRRLVDDADAMEDQDLYEPPASLPKLPGKFLQYTVGGSDPHPGIGHEKDIRQNAVA
LLDQSRRDMFHTVTPSLVFLCLLIPGLHAAFVHGGVPRESYLSTPVTRGEQTVVKTAKFYGEKT
TQRDLTELEISSIFSHCCSLLIGVVIGSSSKIKAGAEQIKKRFKTMMAALNRPSHGETATLLQMFN
PHEAIDWINGQPWVGSFVLSLLTTDFESPGKEFMDQIKLVASYAQMTTYTTIKEYLAECMDATL
TIPVVAYEIRDFLEVSAKLKEEHADLFPFLGAIRHPDAIKLAPRSFPNLASAAFYWSKKENPTMA
GYRASTIQPGASVKETQLARYRRREISRGEDGAELSGEVSAIMKMIGVTGLNLEHHHHHH
**SEQ ID NO 5 (BoDV-1-P (23 kDa, His-Tag))** 
MATRPSSLVDSLEDEEDPQTLRRERSGSPRPRKIPRNALTQPVDQLLKDLRKNPSMISDPDQR
TGREQLSNDELIKKLVTELAENSMIEAEEVRGTLGDISARIEAGFESLSALQVETIQTAQRCDHS
DSIRILGENIKILDRSMKTMMETMKLMMEKVDLLYASTAVGTSAPMLPSHPAPPRIYPQLPSAPT
ADEWDIIPLEHHHHHH
**SEQ ID NO 6 (VSBV-N-Peptid, Aminosäuren 116-130 von A0A0H5BWD6)**
   FVKVS RFYGERTASR
**SEQ ID NO7 (NR1 Untereinheit des NMDAR-Rezeptors)**

MSTMHLLTFALLFSCSFARAACDPKIVNIGAVLSTRKHEQMFREAVNQANKRHGSWKIQLNATS
VTHKPNAIQMALSVCEDLISSQVYAILVSHPPTPNDHFTPTPVSYTAGFYRIPVLGLTTRMSIYSD
KSIHLSFLRTVPPYSHQSSVWFEMMRVYNWNHIILLVSDDHEGRAAQKRLETLLEERESKAEKV
LQFDPGTKNVTALLMEARELEARVIILSASEDDAATVYRAAAMLNMTGSGYVWLVGEREISGNA
LRYAPDGIIGLQLINGKNESAHISDAVGVVAQAVHELLEKENITDPPRGCVGNTNIWKTGPLFKR
VLMSSKYADGVTGRVEFNEDGDRKFANYSIMNLQNRKLVQVGIYNGTHVIPNDRKIIWPGGET
EKPRGYQMSTRLKIVTIHQEPFVYVKPTMSDGTCKEEFTVNGDPVKKVICTGPNDTSPGSPRH
TVPQCCYGFCIDLLIKLARTMNFTYEVHLVADGKFGTQERVNNSNKKEWNGMMGELLSGQAD
MIVAPLTINNERAQYIEFSKPFKYQGLTILVKKEIPRSTLDSFMQPFQSTLWLLVGLSVHVVAVML
YLLDRFSPFGRFKVNSEEEEEDALTLSSAMWFSWGVLLNSGIGEGAPRSFSARILGMVWAGFA
MIIVASYTANLAAFLVLDRPEERITGINDPRLRNPSDKFIYATVKQSSVDIYFRRQVELSTMYRHM
EKHNYESAAEAIQAVRDNKLHAFIWDSAVLEFEASQKCDLVTTGELFFRSGFGIGMRKDSPWK
QNVSLSILKSHENGFMEDLDKTWVRYQECDSRSNAPATLTFENMAGVFMLVAGGIVAGIFLIFIE

IAYKRHKDARRKQMQLAFAAVNVWRKNLQDRKSGRAEPDPKKKATFRAITSTLASSFKRRRSS
KDTSTGGGRGALQNQKDTVLPRRAIEREEGQLQLCSRHRES

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.
**Fig. 1** zeigt den Aufbau eines Lineblot-Streifens mit bornaviralen Antigenen, wie er in Beispiel 1 hergestellt und verwandt wurde.
**Fig. 2** zeigt einen Lineblot-Streifen mit bornaviralen Antigenen, wie er in Beispiel 1 hergestellt wurde, nach der Inkubation gemäß Beispiel 1.
**Fig. 3** zeigt die Ergebnisse der Untersuchung von Proben von Patienten mit limbischer Encephalitis unter Verwendung eines in Beispiel 1 hergestellten Lineblot-Streifens.
**Fig. 4** zeigt die Ergebnisse der Untersuchung von Proben von Patienten mit limbischer Encephalitis unter Verwendung eines weiteren in Beispiel 1 hergestellten Lineblot-Streifens.
**Fig. 5** zeigt das Ergebnis der Untersuchung einer Probe von einem Transplantationspatienten mittels Immunfluoreszenz, siehe Beispiel 2, in zwei unterschiedlichen Vergrößerungen (links 200x, rechts 400x).
**Fig. 6** zeigt die Untersuchung von Proben eines Transplantationspatienten unter Verwendung eines gemäß Beispiel 1 hergestellten Lineblot-Streifens mit bornaviralen Antigenen, siehe Beispiel 2.

### Beispiel 1: Membranbasierter Antikörpernachweis gegen das Variegated Squirrel Bornavirus I (VSBV1) und das Mammalian Bornavirus I (BoDV-11)

### 1.1. Herstellung eines EUROLINE Anti-Bornavirus-Profils Beschichtung der Antigene auf Nitrocellulose-Membran:

VSBV-N-Protein (SEQ ID NO 1), VSBV-P-Peptid (SEQ ID NO 2), BoDV-1-N-Protein (SEQ ID NO 4), BoDV-1-P-Protein (SEQ ID NO 5) und VSBV-P-Protein (SEQ ID NO 3) wurden rekombinant in E. coli mit einem His-Tag fusioniert aufgereinigt und unter Verwendung von Standardprotokollen affinitätschromatographisch aufgereinigt. Die aufgereinigten Proteine wurden gefroren gelagert (-70°C) und vor der Beschichtung aufgetaut.

Für die Herstellung des EUROLINE Anti-Bornavirus-Profil a wurden die Antigene in je zwei Verdünnungen beschichtet (mit pro Antigen variablen Konzentrationen von 9 µg/mL bis 59 µg/mL).

Auf einem folgenden zweiten EUROLINE Anti-Bornavirus-Profil b wurden die Konzentrationen der BoDV-1-Antigene um je 50% verringert. Die Beschichtung der verdünnten Antigene erfolgte maschinell mit einem contact tip dispenser (siehe: Raphael Wong, Harley Tse: Lateral Flow Immunoassay; Humana Press; 2008, S. 137). **Fig. 1** zeigt den Aufbau des EUROLINEs, dabei bezeichnet 1 die höhere Antigenkonzentration und 2 die geringere Antigenkonzentration.

Anschließend wurden die beschichteten Membranen blockiert, gespült, getrocknet und zur weiteren Verwendung bei -20°C gelagert.

### Verkleben der Beschichtungen auf Trägerfolie:

Zum Erstellen des EUROLINE Anti-Bornavirus-Profils wurden Linien von jedem Antigen auf Kunststoff-Folien fixiert. Anschließend erfolgte die Zerteilung in einzelne Teststreifen.

### 1.2. Durchführung von Immunassays unter Verwendung des EUROLINEs Inkubation von Serumproben:

### 1. Vorbehandlung:

Die Inkubationsrinnen wurden entsprechend der Zahl der zu untersuchenden Patientenproben mit je 1,5 mL gebrauchsfertig verdünntem Universalpuffer (ZD 1100-1050, EUROIMMUN AG) gefüllt. Die benötigte Menge an Teststreifen wurden mit einer Pinzette der Verpackung entnommen und direkt in je eine mit Puffer gefüllte Inkubationsrinne gelegt und 15 Minuten bei Raumtemperatur auf einem Wippschüttler inkubiert. Anschließend wurde die Flüssigkeit aus den Rinnen vollständig abgezogen.

### 2. Proben-Inkubation:

In jede mit einem Teststreifen gefüllte Inkubationsrinne wurde 1,5 mL der 1:51 in Universalpuffer verdünnten Patientenprobe gefüllt und 30 Minuten auf einem Wippschüttler bei Raumtemperatur (+23°C) inkubiert.

### 3. Waschen:

Die Flüssigkeit wurde aus jeder Rinne vollständig abgezogen und 3 × 5 Minuten mit je 1,5 mL gebrauchsfertig verdünntem Universalpuffer auf einem Wippschüttler gewaschen.

### 4. Konjugat-Inkubation:

Jeweils 1,5 mL gebrauchsfertig verdünntes Enzymkonjugat (Alkalische-Phosphatasemarkiertes Anti-Human-IgG; AE 142-1030, EUROIMMUN AG) wurde in die Inkubationsrinnen pipettiert und 30 Minuten bei Raumtemperatur (+23°C) auf einem Wippschüttler inkubiert.

### 5. Waschen:

Die Flüssigkeit wurde vollständig aus den Rinnen abgezogen. Schritt 3. wurde erneut durchgeführt.

### 6. Substrat-Inkubation:

Jeweils 1,5 mL Substratlösung (ZW 1020-0130, EUROIMMUN AG) wurde in die Inkubationsrinnen pipettiert. Es folgte eine Inkubation von 10 Minuten bei Raumtemperatur (+23 °C) auf einem Wippschüttler.

### 7. Stoppen:

Die Flüssigkeit aus jeder Rinne wurde vollständig abgezogen und jeder Teststreifen 3 × 1 Minute mit destilliertem oder entionisiertem Wasser gespült.

### 8. Auswerten:

Die Teststreifen wurden luftgetrocknet und ausgewertet.

### Inkubation von Liquor-Proben:

### 1. Vorbehandeln:

Die Inkubationsrinnen wurden entsprechend der Zahl der zu untersuchenden Patientenproben mit je 1,5 mL Universalpuffer gefüllt. Die benötigte Menge an Teststreifen wurde mit einer Pinzette der Verpackung entnommen und direkt in je eine mit Puffer gefüllte Inkubationsrinne gelegt. Es wurde 15 Minuten bei Raumtemperatur (+23 °C) auf einem Wippschüttler inkubiert. Anschließend wurde die Flüssigkeit aus den Rinnen vollständig abgezogen.

### 2. Proben-Inkubation:

In jede mit einem Teststreifen gefüllte Inkubationsrinne wurden 1,0 mL der verdünnten Patientenproben (Verdünnungsfaktor Liquor 1:4, verdünnt in Universalpuffer) gefüllt. Es folgte eine dreistündige Inkubation auf einem Wippschüttler bei Raumtemperatur (+23 °C).

### 3. Waschen:

Die Flüssigkeit wurde aus jeder Rinne vollständig abgezogen und 3 × 5 Minuten mit je 1,5 mL gebrauchsfertig verdünntem Waschpuffer auf einem Wippschüttler gewaschen.

### 4. Konjugat-Inkubation:

Jeweils 1,5 mL gebrauchsfertig verdünntes Enzymkonjugat (Alkalische-Phosphatasemarkiertes Anti-Human-IgG) wurde in die Inkubationsrinnen pipettiert. Es wurde eine Stunde lang bei Raumtemperatur (+23 °C) auf einem Wippschüttler inkubiert.

### 5. Waschen:

Die Flüssigkeit wurde vollständig aus den Rinnen abgezogen und wie oben gewaschen.

### 6. Substrat-Inkubation:

Es wurden jeweils 1,5 mL Substratlösung in die Inkubationsrinnen pipettiert. Es wurde 20 Minuten bei Raumtemperatur (+23 °C) auf einem Wippschüttler inkubiert.

### 7. Stoppen:

Die Flüssigkeit wurde aus jeder Rinne vollständig abgezogen und jeder Teststreifen 3 × 1 Minute mit destilliertem oder entionisiertem Wasser gespült.

### 8. Auswerten:

Die Teststreifen wurden luftgetrocknet und ausgewertet.

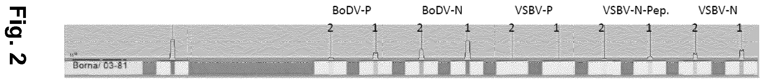

**Fig. 2** zeigt einen beispielhaften Streifen nach der Inkubation.

### Auswertung:

Die mit der EUROLINEScan-Software (EUROIMMUN Medizinische Labordiagnostika AG, Lübeck) gemessenen Intensitäten werden von einem kundigen Mitarbeiter unter Berücksichtigung des unten genannten Kriteriums ausgewertet. Für ein positives Ergebnis muss je die spezifische Antigenbeschichtung (geringer konzentrierte der beiden beschichteten Verdünnungen) oberhalb des Cut-Offs reagieren.

Für das EUROLINE Anti-Bornavirus Profil a wurde der Cut-Off wie folgt festgelegt. Die Zahlen entsprechen den im EUROLINE-Scan gemessenen Intensitäten der Banden:
BoDV-1-Antigene: 0-23 = negativ; 24-30 = grenzwertig; >30 = positiv
VSBV-Antigene: 0-13 = negativ; 14-20 = grenzwertig; >20 = positiv

Für das EUROLINLE Anti-Bornavirus Profil b wurde der folgende Cut-Off festgelegt:
Alle Antigene: 0-13 = negativ; 14-20 = grenzwertig; >20 = positiv

### 1.3. Ergebnisse unter Verwendung verschiedener Probenkollektive: NMDA-R-IFT-vorcharakterisierte Patientenseren:

Es wurden anonymisierte Serumproben von Patienten untersucht, die unter Symptomen von limbischen Encephalitiden leiden.

Insgesamt wurden n=60 in einem ersten Versuch auf dem EUROLINE Anti-Bornavirus-Profil a und weiteren n=49 Proben in einer zweiten Studie auf dem EUROLINE Anti-Bornavirus-Profil b inkubiert, die diesen Anforderungen entsprechen. Die Proben wurden mittels Immunfluoreszenz (Produkt FA 112d-1003-51, EUROIMMUN Medizinische Labordiagnostika AG) auf die Anwesenheit von Autoantikörpern gegen den NMDA-Rezeptor untersucht. Anschließend wurden sie auf dem EUROLINE Anti-Bornavirus-Profil getestet, wie er unter 1.1. hergestellt wurde.

Bei Untersuchung mittels Immunfluoreszenz haben von insgesamt n=109 Proben n=7 Proben positiv in der anti-NMDA-R-IFT reagiert. Bei diesen Patienten ist die Encephalitis also auf Autoantikörper gegen den NMDA-Rezeptor zurückzuführen. Keines der n=7 NMDA-R-IFTpositiven Seren reagierte positiv auf dem EUROLINE Anti-Bornavirus-Profil.

Bei den übrigen n=102 Patienten, deren Proben in deranti-NMDA-R-IFT negativ reagierten, bleibt die Ursache der Erkrankung ungeklärt. Bei diesem Kollektiv sind unter anderem Bornavirus-Infektionen als möglicher Auslöser zu erwarten.

Von diesen n=102 Proben reagierten insgesamt n=14 Proben positiv mit mindestens einem Bornavirus-Antigen (davon n=7 im ersten Versuch und n=7 im zweiten Versuch). Dies entspricht 13,73% der Enzephalitiden, die durch eine Bornavirus-Infektion hervorgerufen werden könnten.

### Seren von Patienten mit Multipler Sklerose

Auch bei Enzephalomyelitis disseminata sind die genauen Ursachen noch unklar. Diese Encephalitis betrifft jedoch einen anderen Bereich des ZNS als eine limbische Encephalitis und dient als Kontrollkollektiv. Es wurden n=36 Proben auf dem EUROLINE Anti-Bornavirus-Profil b getestet, von denen n=1 Serum positiv mit dem EUROLINE Anti-Bornavirus-Profil reagierte.

### Positivkontrollen:

Als Positivkontrollen wurden auf dem EUROLINE Anti-Bornavirus Profil a folgende Proben verwendet:
n=1 anti-VSBV-positiver Liquor
n=1 Serum von einem Patienten mit Psychose
n=2 serologisch vorcharakterisierte BoDV-1-positive Seren

Auf dem EUROLINE Anti-Bornavirus-Profil b wurden folgende Positivkontrollen inkubiert:
n= 4 polyklonale Kaninchenseren (je eines gegen VSBV-N, VSBV-P, BoDV-1-N, BoDV-1-P, hergestellt durch Immunisierung von Kaninchen gemäß Standardprotokollen unter Verwendung von VSBV-N-Protein, VSBV-N-Peptid, BoDV-1-N-Protein, BoDV-1-P-Protein und VSBV-P-Protein mit den in Abschnitt 1.1. des Beispielteils genannten Sequenzen.

Von diesen insgesamt n=8 Seren reagierten alle positiv mit mindestens einem Antigen auf dem EUROLINE Anti-Bornavirus Profil, die polyklonalen Antikörper erkannten je das entsprechende beschichtete Antigen.

### Negativkontrollen:

Es wurden Liquor-Proben eingesetzt, die nicht von Patienten mit vermuteter limbischer Encephalitis stammen. Keine der Liquor-Proben reagierte positiv mit dem EUROLINE Anti-Bornavirus-Profil, was die Spezifität des Forschungsstreifens unter diesen Inkubationsbedingungen zeigt.

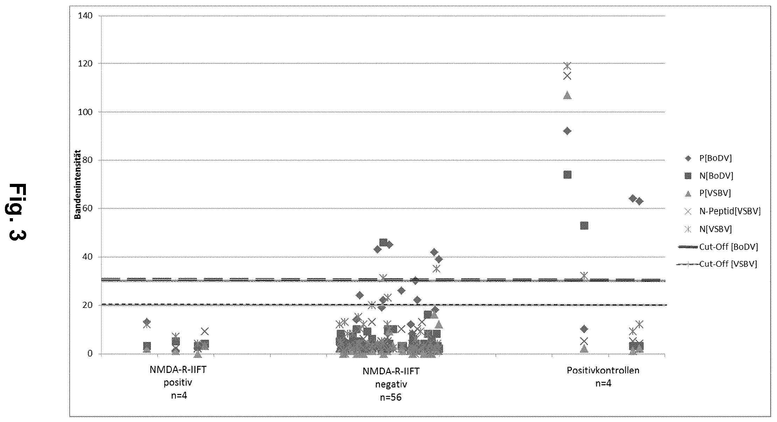

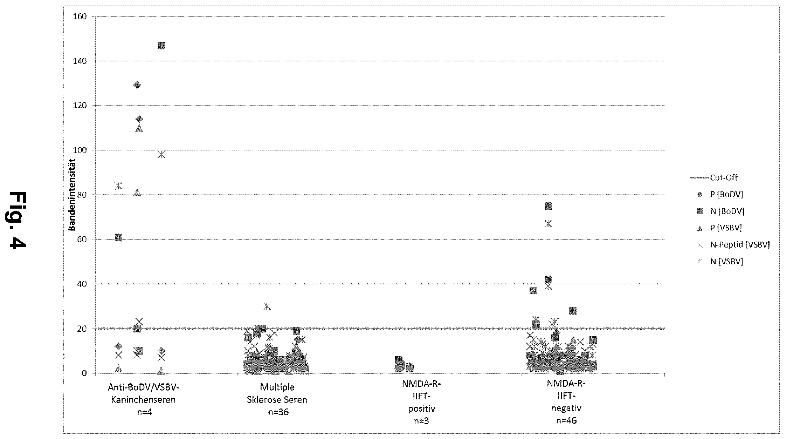

Die Ergebnisse mit dem EUROLINE Anti-Bornavirus Profil a sind in **Fig. 3** dargestellt, die Ergebnisse mit dem EUROLINE Anti-Bornavirus Profil b in **Fig. 4****.** Sie illustrieren, dass kein unter NMDAR-Rezeptor-Encephalitis und assoziiertem PNS bzw. assoziierten Krebsarten leidender Patient Antikörper gegen BoDV-1 oder VSBV-1 aufwies.

### Beispiel 2: Nachweis von Antikörpern gegen Bornavirus in einer Probe von einem Patienten mit post-Transplantations-Komplikationen

### Patienten:

Patient 1 erhielt eine Organspende von einem klinisch unauffälligen Spender. Der Empfänger entwickelte nach der Transplantation neurologische Komplikationen, die in einer Encephalitis kulminierten.

Als Negativkontrollen wurden 264 Blutproben von gesunden Blutspendern untersucht.

### Antikörper-Nachweis:

Serumproben von beiden Patienten wurden unter Verwendung des Anti-Chikungunya-Viren-IIFT der Firma EUROIMMUN Medizinische Labordiagnostika AG untersucht (Bestellnummer FI 293a-1005 G), abgesehen davon, dass als Antigen nicht das im genannten Test eingesetzte (CHIKVinfizierte Vero-Zellen) verwendet wurde, sondern die mit BoDV-1 infizierte Zelllinie CRFK 227, wie sie in Hoffmann et al. (2015) A variegated squirrel bornavirus associated with fatal human encephalitis, N Engl J Med 2015; 373, Seiten 154-162, und im dazugehörigen Supplementary Appendix (S. 19) beschrieben ist. Es wurde das Protokoll in der Anleitung des Herstellers befolgt.

Regelmäßig ab dem Tag 72 nach der Transplantation entnommene Proben wurden auf die Anwesenheit von Antikörpern gegen BoDV-1-P, BoDV-1-N, VSBV-P und VSBV-N untersucht. Dabei wurden die in Beispiel 1 beschriebenen Blots verwendet und der Nachweis wie im genannten Beispiel durchgeführt.

### Ergebnisse:

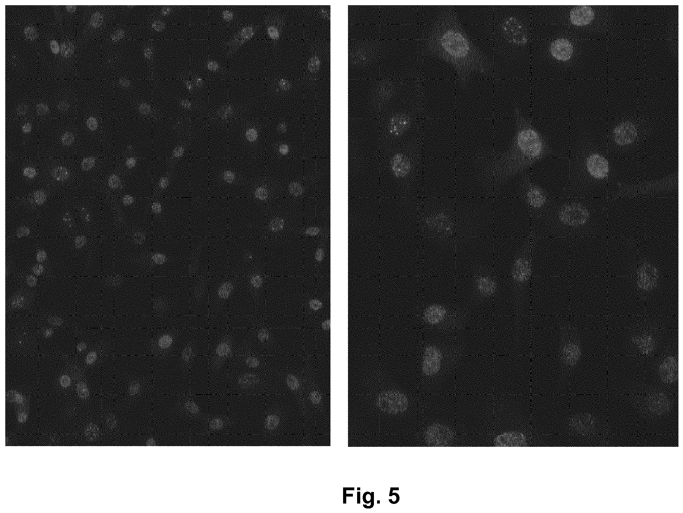

Ein Teil der Folgeproben des Patienten 1 wurden mit dem indirekten Immunfluoreszenztest untersucht. Alle Proben zeigten eine positive Reaktivität, d.h. in den Bornavirus-infizierten Zellen war das spezifische Fluoreszenzmuster erkennbar. Dieses ist beispielhaft in **Fig. 5** dargestellt. In den Zellkernen der infizierten Zellen fluoreszieren einzelne bis mehrere Granula, die das Virusantigen enthalten. Dies bestätigt die Anwesenheit von Bornavirus-spezifischen Antikörpern in der Patientenprobe.

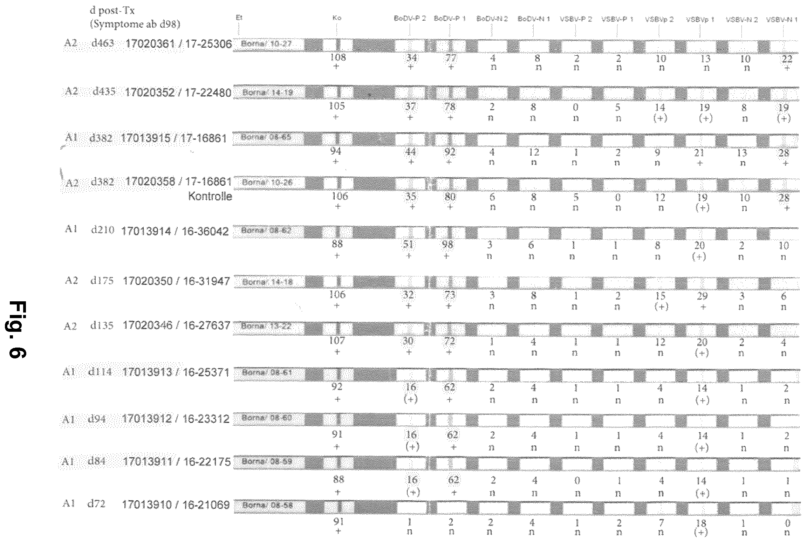

Die Untersuchung der Blutproben im Lineblot zeigte, dass am Tag 72 nach der Transplantation noch keine Antikörper nachweisbar waren (**Fig. 6**). Ab dem Tag 84 waren Antikörper gegen BoDV-1-P nachweisbar, ab dem Tag 382 auch gegen VSBV-N. 98,5 % der Blutspender waren gemäß IIFT negativ.

### Schlussfolgerung

Es kann daher die Schlussfolgerung gezogen werden, dass der Organspender latent mit dem BoDV-1 infiziert war, ohne dass es zu einer klinisch apparenten Infektion kam. Die Transplantation bedingte einen Ausbruch der Infektion in den Organempfängern, vermutlich bedingt durch die immunsuppressive Behandlung.

### Beispiel 3: Limbische Encephalitis durch Infektion mit VSBV-1

### Methoden

### Fallstudie

Im Juli 2013 entwickelte eine 45-jährige Tierpflegerin aus Schleswig-Holstein Fieber, Dysphonie, Husten, Pharyngitis, Vertigo und Parästhesie unter ihrem Auge. Diesen Symptomen folgten Ataxie, Koma und Insuffizienz der Hypophysendrüse. Die Patientin hatte keine bekannten Vorerkrankungen. Analyse des CSF hatte eine lymphozytische Pleozytose gezeigt. Die Entzündungsparameter im peripheren Blut waren erhöht, mit relativer Neutrophilie und Lymphopenie. Eine anfängliche MRI-Untersuchung des Kopfes zeigte normale Ergebnisse. Eine Nachfolgeuntersuchung mittels MRI 3 Wochen später zeigte Läsionen mit einer bilateralen limbischen Verteilung (mediale temporale Lappen, anteriores Cingulum, Insula, Hippocampus, Hypothalamus, periventrikuläres Tectum), in den Basalganglia und im oberen Myelon. Morphologisch wurde limbische Encephalitis mit Fortschreiten innerhalb von einer Woche diagnostiziert. Umfangreiche Laboruntersuchungen mit Hinblick auf Infektionen des zentralen Nervensystems und Autoimmunkrankheiten zeigten normale Ergebnisse. Es wurde keine zu Grunde liegende Neoplasie entdeckt. Wiederholte Elektroenzephalogramme zeigten allgemeine langsame Aktivität und nicht konvulsive epileptische Anfälle. Es wurden keine neurotropischen Bakterien, Pilze, Parasiten oder Viren mittels Mikroskopie, Kultur oder PCR gezeigt. Durch Immunhistochemie konnte eine Creutzfeldt-Jakob Erkrankung ausgeschlossen werden. Aufgrund von bilateraler Lungenentzündung benötigte die Patientin künstliche Beatmung, und sie wurde mit einer breiten Antiinfektionstherapie (umfassend Acyclovir), Antikonvulsanzien und mit Steroiden im späteren Verlauf der Krankheit behandelt. Sie starb schließlich an Myelo-Encephalitis einer zum damaligen Zeitpunkt unbestimmten Ätiologie innerhalb von 3 Monaten nach Beginn der Symptome.

### PCR

Für die Analysen stand eingelagertes gefrorenes CSF und formalinfixiertes, in Paraffin eingebettetes Gehirngewebe, Myokardium, Lungengewebe, Nierengewebe, Lebergewebe, Milzgewebe, Pankreasgewebe, Knochenmark und Eingeweidegewebe für die Analysen zur Verfügung. Eine VSBV-1 spezifische Realtime-RT-PCR wurde wie in Hoffmann *et al.* (2015) beschrieben durchgeführt.

### Immunhistochemie

Polyklonale Antisera gegen N- und P-Proteine von VSBV-1 und BoDV-1 wurden aus Kaninchen erhalten, die mit dem jeweiligen rekombinanten Antigen immunisiert worden waren, und sie wurden mittels lonenaustauschchromatographie über Protein A aufgereinigt (Davids Biotechnologie, Regensburg, Deutschland). Die Reaktivität der Kaninchen-Antisera und Präimmun-Seren wurde mittels Immunfluoreszenz- Antikörpertest (IFAT) und mithilfe des in Beispiel 1 beschriebenen Bornavirus-Immunblots getestet. Zehn nicht verwandte menschliche Gehirngewebeproben wurden als Negativkontrollen eingesetzt. Nach Vorbehandlung mit Proteinase K und Blockierung endogener Peroxidase wurden die FFPE-Schnitte der Patienten mit den Antisera inkubiert (1:1,000-1:5,000 in PBS, über Nacht bei Raumtemperatur). Anschließend wurden sie mit einem Ziegen Anti-Kaninchen biotinylierten Polymer Antikörper, einem Streptavidin-Meerrettich-Peroxidase-Komplex und mit dem 3-Aminum-9-Ethylcarbazol (AEC) Substrat (DCS, Hamburg, Deutschland) inkubiert.

### Serologische Tests

Zum Nachweis Bornavirus-spezifischer IgG-Antikörper im Serum und CSF, wurde eine dauerhaft mit BoDV-1 infizierte Zelllinie für einen standardgemäßen indirekten Immunfluoreszenztest (IFAT) eingesetzt (Hoffmann *et al.,* 2015); zusätzlich wurde ein ELISA und ein Immunblot entwickelt.

Für den VSBV-1-IgG ELISA wurden die Sequenzen von VSBV-1 N- und P-Protein kloniert und in Form von Fusionsproteinen mit Maltose bindendem Protein (MBP) im *E. coli*-Stamm BL21 GOLD (DE3) (Novagen-Merck, Darmstadt, Deutschland) exprimiert. Das Protein wurde über Amylose-Affinitätschromatographie aufgereinigt und eluiert. Das N-terminale MBP-Tag wurde über 3C-Protease bei 4°C über Nacht abgespalten. Nach Entfernung der Protease wurde die Proteinprobe über Gelfiltrations-Chromatografie weiter aufgereinigt (Superdex 200, Sigma-Aldrich, München, Deutschland). Mikrotiterplatten (Polysorp, Nunc, Roskilde, Dänemark) wurden über Nacht bei 4°C mit 2 µg/mL VSBV-1-N- oder P-Protein beschichtet. Nach dem Blockieren mit 6% BSA wurde menschliches Serum verdünnt in 1% BSA- Verdünnungslösung hinzugefügt. Nach zweistündiger Inkubation bei 37°C wurde 100 µl antihumane IgG (Dako Cytomation, Hamburg, Deutschland, 1:6,000 verdünnt), und die Platten wurden 37°C eine Stunde lang inkubiert. Die Reaktion wurde nach 5 Minuten Inkubation bei Raumtemperatur mit 3,3',5,5'- Tetramethylbenzidin gestoppt. Schließlich wurde die optische Dichte (OD) bei 450 nm gemessen (Referenz: 620 nm). Der finale OD-Wert für jede Serumprobe wurde als Differenz zwischen den VSBV-1- N- oder P-Protein enthaltenden und den MBP enthaltenden Wells gemessen. Die finalen OD Werte für Serumverdünnung von 1:400 wurden als positiv betrachtet, wenn der gemittelte OD den gemittelten OD plus dreifacher Standardabweichung überstieg, die mit Kontrollproben von 200 gesunden Blutspendern erhalten wurden.

Für den Bornavirus-IgG-Immunblot wurden die in Beispiel 1 hergestellten Streifen eingesetzt. Sie wurden bei Raumtemperatur mit Serum inkubiert (30 Minuten, 1:51) oder mit CSF (3 Stunden, 1:4), gefolgt von einer Inkubation mit Konjugat von alkalischer Phosphatase (30 min oder 1 Stunde, 1:10 jeweils), und Nitro-Blau-Tetrazoliumchlorid als Substrat (10 oder 20 min). Die Intensitäten der nachgewiesenen Antikörper wurden automatisch unter Verwendung der EurolineScan-Software (EUROIMMUN AG, Lübeck, Deutschland) ausgewertet. Für die Validierung wurden Proben von 150 gesunden Blutspendern getestet.

### Ergebnisse

### Serologie mit Proben der Encephalitis-Patientin

Für Bornavirus spezifische IgG-Antikörper wurden in hohen Konzentrationen im CSF der Patientin in Form eines nukleären Musters durch Immunfluoreszenz nachgewiesen (IgG Endpunkt-Titer 1:2560, und durch ELISA. Eine starke IgG-Reaktivität wurde auf dem Immunblot gegen VSBV-1-N-Polypeptid gezeigt, und in einem geringeren Ausmaß gegen VSBV-1-P- und BoDV-1-P-Antigene (**Tab. 1**).

**Tab. 1 : Serologische Testergebnisse der Encephalitis-Patientin und der Tierpfleger.**

| **Kategorie** | | **Alter** | **Geschlecht** | **IgG Immunoblot*** | | | | **BoDV-1 IgG IFAT** | **VSBV-1 IgG ELISA^{§}** | | **Kontakt mit Bunthörnchen** |
|---|---|---|---|---|---|---|---|---|---|---|---|
| | | | | **BoDV- 1 -P** | **BoDV -1 -N** | **VSBV -P** | **VSBV -N** | | **VSBV -P** | **VSBV -N** | |
| **Encephalitis Patient (CSF)** | | 45 | W | + | o | + | + | **1:2,560** | **pos** | **pos** | regelmäßig |
| **14 Tierpfleger (Serum)** | | 44 | W | o | o | o | **(+)** | neg | neg | neg | regelmäßig |
| | | 32 | M | o | o | o | o | neg | neg | neg | regelmäßig |
| | | 25 | W | o | + | o | **(+)** | neg | neg | neg | selten |
| | | 33 | W | o | o | o | o | neg | neg | neg | gelegentlich |
| | | 26 | W | o | o | o | **(+)** | **1:160^{#}** | neg | neg | gelegentlich |
| | | 27 | M | o | **(+)** | o | o | neg | neg | neg | gelegentlich |
| | | 29 | W | o | o | o | o | neg | neg | neg | selten |
| | | 48 | W | o | **(+)** | o | **(+)** | **1:40** | neg | neg | selten |
| | | 35 | M | o | o | o | o | neg | neg | neg | gelegentlich |
| | | 24 | W | o | o | o | o | neg | neg | neg | regelmäßig |
| | | 18 | W | o | + | o | o | neg | neg | neg | regelmäßig |
| | | 21 | W | o | o | o | **(+)** | neg | neg | neg | gelegentlich |
| | | 37 | M | o | o | o | **(+)** | neg | neg | neg | regelmäßig |
| | | 20 | W | o | o | o | o | neg | neg | neg | regelmäßig |
| **150 Gesunde Blutspender (Serum)** | positiv | N/A | N/A | 1.5% | 0% | 1.5% | 1.5% | N/A | neg | neg | kein |
| | schwach positiv | | | 4.5% | 4.5% | 0% | 12% | | | | |

| | | | | | | | | | | | |
|---|---|---|---|---|---|---|---|---|---|---|---|
| BoDV-1, Bornakrankheitvirus; CSF, Liquor; ELISA; enzyme-linked immunosorbent assay, IFAT immunofluoreszenz-Antikörpertest; VSBV-1, Bunthörnchen Bornavirus-1; N/A nicht verfügbar. +, stark positiv; (+) schwach positiv; o keine Reaktion im Immunblot. * Die Ergebnisse wurden unter Verwendung der EUROLineScan-Software densitometrisch erhalten und in semiquantitative Kategorien überführt (negativ, o: 0-13, schwach positiv, (+): 14 - 20; positiv, +: 21-255). § Finale OD-Werte für Serumverdünnung von 1:400 wurden als positiv betrachtet, wenn die mittlere OD die mittlere OD plus dreifache Standard Abweichung erhalten mit negativen Kontrollproben überstieg. # Der IgG-Endpunkt Titer wird definiert als die reziproke höchste Analytenverdünnung, die in der Immunfluoreszenz ein positives Signal ergibt. | | | | | | | | | | | |

### Serologische Untersuchung der Tierpfleger

Seren von allen vierzehn Tierpflegern wurden unter Verwendung von ELISA, Immunfluoreszenz und Immunblot untersucht. Sechs Tierpfleger hatten regelmäßig Kontakt mit den Bunthörnchen, und fünf von ihnen regelmäßig. Die übrigen Personen hatten nur selten Kontakt. Dem Immunblot zu urteilen reagierten sechs der vierzehn Seren positiv oder schwach positiv mit VSBV-1-N und vier mit BoDV-1-N, aber keines mit dem entsprechenden P-Antigen. Der Immunfluoreszenztest zeigte geringe Titer in zwei Fällen, wohingegen ELISA-Ergebnisse negativ waren. Die serologische Reaktivität korrelierte nicht mit der berichteten Intensität des Kontaktes mit den Hörnchen. Gemäß Immunblot reagierten 13,5% der Blutspender positiv oder schwach positiv mit VSBV-1-N, 4,5% mit BoDV-1-N, 1,5% mit VSBV-1-P und 6% mit BoDV-1-P-Antigen. Einer der Blutspender reagierte mit mehr als einem Bornavirus-Antigen.

## Patentansprüche

1. Verfahren zur Diagnose einer limbischen Encephalitis, PNS, Encephaloymyelitis, Leukoencephalopathie, Retinitis oder Optikusatrophie umfassend den Schritt Nachweisen eines Mammalian 2 Bornavirus VSBV-1, und umfassend den Schritt Nachweisen eines Mammalian 1 Bornavirus BoDV-1 in einer menschlichen Probe, wobei der jeweilige Nachweis durch Detektieren eines Antikörpers gegen das jeweilige Bornavirus-spezifische Polypeptid in der Probe erfolgt.

2. Verfahren zum Prognostizieren von neurologischen post-Transplantations-Komplikationen oder zum Screenen von Organspendern oder Spenderorganen, umfassend den Schritt Nachweisen eines Bornavirus in einer menschlichen Probe.

3. Verfahren zur Differenzierung einer Autoimmun- und einer infektionsbedingten Encephalitis, umfassend den Schritt Nachweisen eines Bornavirus in einer menschlichen Probe.

4. Verfahren nach einem der Ansprüche 2 bis 3, wobei der Nachweis durch Detektieren eines Antikörpers gegen ein Bornavirus-spezifisches Polypeptid in der Probe erfolgt, bevorzugt aus der Gruppe umfassend ein N-, P- und X-Protein des Bornavirus.

5. Verfahren nach Anspruch 4, wobei die Probe mit einem Träger kontaktiert wird, der ein Mittel zum Einfangen eines Antikörpers gegen wenigstens ein Polypeptid umfasst, das aus der Gruppe ausgewählt ist, die BoDV-1-N, BoDV-1-P, BoDV-1-X, VSBV-N, VSBV-P und VSBV-X umfasst.

6. Verfahren nach Anspruch 5, wobei der Träger ausgewählt ist aus der Gruppe umfassend einen membranbasierten Immunblot, bevorzugt ein Western-Blot oder ein Line-Blot, ein oder mehr als ein Bead, einen für Immunfluoreszenz hergerichteten Biochip und eine Mikrotiterplatte für ELISA.

7. Träger umfassend ein Mittel zum Einfangen eines Antikörpers gegen wenigstens ein Polypeptid, das aus der Gruppe ausgewählt ist, die BoDV-1-N, BoDV-1-P, BoDV-1-X, VSBV-N, VSBV-P und VSBV-X umfasst, wobei der Träger weiter ein Mittel umfasst zum Einfangen eines Antikörpers gegen ein neurologisches Antigen aus der Gruppe umfassend NMDAR, AMPAR, GAD65, Amphiphysin, GABA(A), GABA(B), DPPX, Lgl1, CASPR2, IGLON5, SNARE, NBC1, Flotillin und ITPR, bevorzugt NMDAR.

8. Verfahren oder Träger gemäß einem der Ansprüche 5 bis 7, wobei der Träger weiter ein Mittel umfasst zum Einfangen eines Antikörpers gegen wenigstens ein Virus aus der Gruppe umfassend Herpex simplex HSV-1, Herpes simplex HSV-2, Humanes Herpesvirus HHV-6, Humanes Herpesvirus HHV-7, Tollwut-Virus, LCMV, West-Nil-Virus, Tick-Borne Encephalitis-Virus, Usutu-Virus, Toscana-Virus, Varizella-Zoster-Virus, Epstein-Barr-Virus, Cytomegalovirus, Equine Encephalitis-Viren, Chikungunya-Virus, La Crosse-Virus, Rift-Tal-Fieber-Virus, St. Louis-Encephalitis-Virus, Influenza-A-Virus, Masernvirus, Mumpsvirus, Rötelnvirus, Hendra-Virus, Nipah-Virus, Enterovirus, California Encephalitis Virus, Powassan-Virus, Murray-Valley-Encephalitis-Virus und Japanische-Encephalitis-Virus.

9. Kit umfassend den Träger nach einem der Ansprüche 7 bis 8 sowie ein Mittel zum Nachweis eines eingefangenen Antikörpers oder eine Kontrolle zum Nachweis der Anwesenheit einer Probe, bevorzugt einer Serum- oder CSF-Probe.

10. In-Vitro-Verwendung eines Trägers umfassend ein Mittel zum Einfangen eines Antikörpers gegen wenigstens ein Polypeptid, das aus der Gruppe ausgewählt ist, die BoDV-1-N, BoDV-1-P, BoDV-1-X umfasst, und umfassend ein Mittel zum Einfangen eines Antikörpers gegen wenigstens ein anderes Polypeptid, das aus der Gruppe ausgewählt ist, die VSBV-N, VSBV-P und VSBV-X umfasst zur Diagnose einer Encephalitis, PNS, Encephaloymyelitis, Leukoencephalopathie, Retinitis oder Optikusatrophie bei einem Menschen.

11. *In-Vitro*-Verwendung eines Trägers umfassend ein Mittel zum Einfangen eines Antikörpers gegen wenigstens ein Polypeptid, das aus der Gruppe ausgewählt ist, die BoDV-1-N, BoDV-1-P, BoDV-1-X, VSBV-N, VSBV-P und VSBV-X umfasst, oder eines zum Nachweis eines Bornavirus geeigneten Primers oder einer ebensolchen Sonde zum Prognostizieren von post-Transplantations-Komplikationen bei einem Menschen, zur Überwachung einer Therapie einer Encephalitis oder von post-Transplantations-Komplikationen bei einem Menschen oder zur Differenzierung einer Autoimmun- und einer infektionsbedingten Encephalitis bei einem Menschen.

12. *In-Vitro*-Verwendung eines Trägers oder Kits nach einem der Ansprüche 7 bis 9 zur Differenzierung einer Autoimmun- und einer infektionsbedingten Encephalitis.

13. Verfahren zur Diagnose einer limbischen Encephalitis umfassend den Schritt Nachweisen eines Bornavirus, bevorzugt aus der Gruppe umfassend Mammalian 2 Bornavirus, noch bevorzugter VSBV-1, und Mammalian 1 Bornavirus, noch bevorzugter BoDV-1, in einer menschlichen Probe.

## Claims

1. Method for diagnosing a limbic encephalitis, PNS, encephaloymyelitis, leukoencephalopathy, retinitis or optic atrophy, comprising the step of detecting a mammalian 2 bornavirus VSBV-1, and comprising the step of detecting a mammalian 1 bornavirus BoDV-1 in a human sample, wherein the respective detection is achieved by detecting an antibody against the respective bornavirus-specific polypeptide in the sample.

2. Method for prognosticating neurological post-transplantation complications or for screening organ donors or donor organs, comprising the step of detecting a bornavirus in a human sample.

3. Method for differentiating between an autoimmune encephalitis and an encephalitis caused by infection, comprising the step of detecting a bornavirus in a human sample.

4. Method according to any of claims 2 to 3, wherein the detection is achieved by detecting an antibody against a bornavirus-specific polypeptide in the sample, preferably from the group comprising an N, P and X protein of the bornavirus.

5. Method according to claim 4, wherein the sample is contacted with a carrier comprising a means for the capture of an antibody against at least one polypeptide selected from the group comprising BoDV-1-N, BoDV-1-P, BoDV-1-X, VSBV-N, VSBV-P and VSBV-X.

6. Method according to claim 5, wherein the carrier is selected from the group comprising a membrane-based immunoblot, preferably a Western blot or a line blot, one or more than one bead, a biochip arranged for immunofluorescence, and an ELISA microtiter plate.

7. Carrier comprising a means for the capture of an antibody against at least one polypeptide selected from the group comprising BoDV-1-N, BoDV-1-P, BoDV-1-X, VSBV-N, VSBV-P and VSBV-X, wherein the carrier further comprises a means for the capture of an antibody against a neurological antigen from the group comprising NMDAR, AMPAR, GAD65, amphiphysin, GABA(A), GABA(B), DPPX, Lgl1, CASPR2, IGLON5, SNARE, NBC1, flotillin and ITPR, preferably NMDAR.

8. Method or carrier according to any of claims 5 to 7, wherein the carrier further comprises a means for the capture of an antibody against at least one virus selected from the group comprising herpex simplex HSV-1, herpes simplex HSV-2, human herpesvirus HHV-6, human herpesvirus HHV-7, rabies virus, LCMV, West Nile virus, tick-borne encephalitis virus, Usutu virus, Toscana virus, varicella zoster virus, Epstein-Barr virus, cytomegalovirus, equine encephalitis viruses, chikungunya virus, La Crosse virus, Rift Valley fever virus, St. Louis encephalitis virus, influenza A virus, measles virus, mumps virus, rubella virus, Hendra virus, Nipah virus, enterovirus, California encephalitis virus, Powassan virus, Murray Valley encephalitis virus and Japanese encephalitis virus.

9. Kit comprising the carrier according to any of claims 7 to 8 and also a means for the detection of a captured antibody or a control for the detection of the presence of a sample, preferably a serum or CSF sample.

10. *In vitro* use of a carrier comprising a means for the capture of an antibody against at least one polypeptide selected from the group comprising BoDV-1-N, BoDV-1-P, BoDV-1-X, and comprising a means for the capture of an antibody against at least one other polypeptide selected from the group comprising VSBV-N, VSBV-P and VSBV-X, for the diagnosis of an encephalitis, PNS, encephalomyelitis, leukoencephalopathy, retinitis or optic atrophy in a human.

11. *In vitro* use of a carrier comprising a means for the capture of an antibody against at least one polypeptide selected from the group comprising BoDV-1-N, BoDV-1-P, BoDV-1-X, VSBV-N, VSBV-P and VSBV-X or a primer suitable for the detection of a bornavirus or just such a probe for the prognostication of post-transplantation complications in a human, for the monitoring of a therapy of an encephalitis or of post-transplantation complications in a human or for the differentiation between an autoimmune encephalitis and an encephalitis caused by infection in a human.

12. *In vitro* use of a carrier or a kit according to any of claims 7 to 9 for the differentiation between an autoimmune encephalitis and an encephalitis caused by infection.

13. Method for diagnosing a limbic encephalitis comprising the step of detecting a bornavirus, preferably from the group comprising mammalian 2 bornavirus, even more preferably VSBV-1, and mammalian 1 bornavirus, even more preferably BoDV-1, in a human sample.

## Revendications

1. Procédé de diagnostic d'une encéphalite limbique, d'une SNP, d'une encéphalomyélite, d'une leucoencéphalopathie, d'une rétinite ou d'une atrophie optique, comprenant l'étape de détection d'un bornavirus mammalien 2 VSBV-1, et comprenant l'étape de détection d'un bornavirus mammalien 1 BoDV-1 dans un échantillon humain, la détection respective étant effectuée par détection d'un anticorps contre le polypeptide spécifique du bornavirus respectif dans l'échantillon.

2. Procédé de pronostic de complications neurologiques post-transplantation ou de dépistage de donneurs d'organes ou des organes de donneurs, comprenant l'étape consistant à détecter un bornavirus dans un échantillon humain.

3. Procédé pour différencier une encéphalite auto-immune d'une encéphalite liée à une infection, comprenant l'étape de détection d'un bornavirus dans un échantillon humain.

4. Procédé selon l'une quelconque des revendications 2 à 3, dans lequel la détection est effectuée en détectant un anticorps contre un polypeptide spécifique du bornavirus dans l'échantillon, de préférence choisi dans le groupe comprenant une protéine N, P et X du bornavirus.

5. Procédé selon la revendication 4, dans lequel l'échantillon est mis en contact avec un support comprenant un moyen de piégeage d'un anticorps contre au moins un polypeptide choisi dans le groupe comprenant BoDV-1-N, BoDV-1-P, BoDV-1-X, VSBV-N, VSBV-P et VSBV-X.

6. Procédé selon la revendication 5, dans lequel le support est choisi dans le groupe comprenant un immunotransfert à base de membrane, de préférence un transfert western ou un line blot, une ou plusieurs billes, une biopuce préparée pour l'immunofluorescence et une plaque de microtitration pour ELISA.

7. Support comprenant un moyen de piéger un anticorps contre au moins un polypeptide choisi dans le groupe comprenant BoDV-1-N, BoDV-1-P, BoDV-1-X, VSBV-N, VSBV-P et VSBV-X, dans lequel le support comprend en outre un agent pour piéger un anticorps contre un antigène neurologique choisi dans le groupe comprenant NMDAR, AMPAR, GAD65, amphiphysine, GABA(A), GABA(B), DPPX, Lgl1, CASPR2, IGLON5, SNARE, NBC1, flotilline et ITPR, de préférence NMDAR.

8. Procédé ou support selon l'une quelconque des revendications 5 à 7, dans lequel le support comprend en outre un moyen pour piéger un anticorps contre au moins un virus choisi dans le groupe comprenant Herpex simplex HSV-1, Herpes simplex HSV-2, Herpesvirus humain HHV-6, Herpesvirus humain HHV-7, Virus de la rage, LCMV, Virus du Nil occidental, virus de l'encéphalite transmise par les tiques, virus de l'Usutu, virus de la Toscane, virus de la varicelle-zona, virus d'Epstein-Barr, cytomégalovirus, virus de l'encéphalite équine, virus du chikungunya, virus de La Crosse, virus de la fièvre de la vallée du Rift, virus de l'encéphalite de St. Louis, virus de la grippe A, virus de la rougeole, virus des oreillons, virus de la rubéole, virus Hendra, virus Nipah, l'entérovirus, virus de l'encéphalite californienne, virus Powassan, virus de l'encéphalite de Murray Valley et virus de l'encéphalite japonaise.

9. Kit comprenant le support selon l'une quelconque des revendications 7 à 8 ainsi qu'un moyen de détection d'un anticorps capturé ou un contrôle pour détecter la présence d'un échantillon, de préférence un échantillon de sérum ou de CSF.

10. Utilisation *in vitro* d'un support comprenant un moyen de piéger un anticorps contre au moins un polypeptide qui est choisi dans le groupe qui comprend BoDV-1-N, BoDV-1-P, BoDV-1-X, et comprenant un moyen pour piéger un anticorps contre au moins un autre polypeptide qui est choisi dans le groupe qui comprend VSBV-N, VSBV-P et VSBV-X, pour le diagnostic d'une encéphalite, d'une SNP, d'une encéphalomyélite, d'une leucoencéphalopathie, d'une rétinite ou d'une atrophie optique chez un être humain.

11. Utilisation *in vitro* d'un support comprenant un moyen de piéger un anticorps contre au moins un polypeptide qui est choisi dans le groupe qui comprend BoDV-1-N, BoDV-1-P, BoDV-1-X, VSBV-N, VSBV-P et VSBV-X, ou une amorce ou une sonde appropriée pour la détection d'un bornavirus, pour le pronostic de complications post-transplantation chez un être humain, pour surveiller le traitement d'une encéphalite ou de complications post-transplantation chez un être humain ou pour différencier une encéphalite auto-immune d'une encéphalite infectieuse chez un être humain.

12. Utilisation *n vitro* d'un support ou d'un kit selon l'une quelconque des revendications 7 à 9 pour différencier une encéphalite auto-immune d'une encéphalite infectieuse.

13. Procédé de diagnostic d'une encéphalite limbique comprenant l'étape consistant à détecter un bornavirus, de préférence choisi dans le groupe comprenant le bornavirus mammalien 2, de préférence encore le VSBV-1, et le bornavirus mammalien 1, de préférence encore le BoDV-1, dans un échantillon humain.
